# EUROPEAN PATENT SPECIFICATION

(11) **EP 2 187 967 B1**
(45) Date of publication and mention of the grant of the patent: **24.04.2013**
(21) Application number: 08797814.4
(22) Date of filing: 13.08.2008
(51) Int. Cl.: A61K 45/06, A61P 35/00

(54) **METHODS AND COMPOSITIONS FOR TREATING CANCERS**
VERFAHREN UND ZUSAMMENSETZUNGEN ZUR BEHANDLUNG VON KREBS
PROCÉDÉS ET COMPOSITIONS POUR TRAITER DES CANCERS

(30) Priority: 16.08.2007 US 956295 P
(43) Date of publication of application: 26.05.2010
(73) Proprietor: IRM LLC, Hamilton HM LX (BM)
(72) Inventor: DIERKS, Christine, 79111 Freiburg (DE); WARMUTH, Markus, Natick, Massachusetts 01760 (US)
(74) Representative: Roth, Peter Richard
(86) International application number: PCT/US2008/073049
(87) International publication number: WO 2009/026075

(56) References cited:
- WO-A-2006/102611
- GALMOZZI E ET AL: "Cancer stem cells and therapeutic perspectives" CURRENT MEDICINAL CHEMISTRY, BENTHAM SCIENCE PUBLISHERS BV, BE, vol. 13, no. 6, 1 January 2006 (2006-01-01), pages 603-607, XP009107987 ISSN: 0929-8673

## Description

### Technical Field

The present invention generally relates to methods for inhibiting tumor cell growth and for treating cancer.

### Background Art

The Hh signaling pathway has been well characterized in the art (see, e.g., Nybakken et al., Curr. Opin. Genet. Dev. 2002, 12:503-511; and Lum et al., Science 2003, 299: 2039-2045). Briefly, in the absence of hedgehog ligands, the transmembrane receptor, Patched (Ptch), binds to Smoothened (Smo) and blocks Smo's function. This inhibition is relieved in the presence of ligands, which allows Smo to initiate a signaling cascade that results in the release of transcription factors Glis from cytoplasmic proteins fused (Fu) and Suppressor of Fused (SuFu). In the inactive situation, SuFu prevents Glis from translocating to the nucleus. In the active situation, Fu inhibits SuFu and Glis are released. Gli proteins translocate into the nucleus and control target gene transcription.

The BCR-ABL oncogene is the product of Philadelphia chromosome (Ph) 22q, and encodes a chimeric BCR-ABL protein that has constitutively activated ABL tyrosine kinase activity. (Lugo et al., Science 1990, 247:1079-1082). BCR-ABL is the underlying cause of chronic myeloid leukemia. Whereas the 210 kDa BCR-ABL protein is expressed in patients with CML, a 190 kDa BCR-ABL protein resulting from an alternative breakpoint in the BCR gene is expressed in patients with Ph positive (Ph⁺) acute lymphoblastic leukemia (ALL). (Bartram et al., Nature 1983, 306:277-280; Chan et al., Nature 1987, 325:635-637).

BCR-ABL has been shown to induce proliferation and anti-apoptosis through various mechanisms in committed myeloid or lymphoid progenitors or 3T3 fibroblasts. (Pendergast et al., Cell 1993, 75:175-85; Ilaria et al., J. Biol. Chem. 1996, 271:31704-10; Chai et al., J. Immunol. 1997, 159:4720-8; and Skorski et al., EMBO J. 1997, 16:6151-61). However, little is known about the effect of BCR-ABL on the hematopoietic stem cell (HSC) population. Recent publications suggest that developmental pathways like the Wnt signaling pathway or the Polycomb gene BMI1 might be involved in the regulation and expansion of leukemic stem cells (Mohty et al., Blood, 2007; Hosen et al., Stem Cells, 2007). BMI1 and beta-catenin are both upregulated in CML blast crisis and their expression correlates with the progression of the disease. BCR-ABL positive granulocyte-macrophage progenitors that have acquired β-catenin expression are candidate leukemic stem cells in blast-crisis CML. The self-renewal pathways involved in the expansion of the BCR-ABL positive leukemic stem cell during chronic phase, which lead to the initial expansion of the malignant clone, are currently not well understood. WO 2006/102611 relates to PP2A activating compounds, Current Med. Chem. 13(6), pages 603-607 mentions cyclopamine as hedgehog signalling inhibitor, but both publications are silent on combinations with two different agents.

### Disclosure of the Invention

The invention provides compositions and pharmaceutical compositions thereof, which may be useful for inhibiting tumor cell growth and for treating a variety of cancers.

In one aspect, the present invention provides a composition as defined in claim 2 and its dependent claims which all are incorporated here by reference, comprising a first agent that inhibits hedgehog signaling pathway and a second agent that inhibits BCR-ABL. In another aspect, the invention provides pharmaceutical compositions comprising a therapeutically effective amount of a first agent that inhibits hedgehog signaling pathway, a second agent that inhibits BCR-ABL, and a pharmaceutically acceptable carrier, each as defined in claim 2 and its dependent claims which all are incorporated here by reference.

In another aspect, the invention provides a combination of a first agent that inhibits hedgehog signalling pathway and a second agent that inhibits BCR-Abl, each as defined in claim 1 or its dependent claims which are incorporated here by reference. An invention embodiment also relates to combinations or compositions for use according to claims 6 or 7 which are also incorporated here by reference. The invention also provides methods for treating cancers, particularly a BCR-ABL positive leukemia, comprising administering to a system or a subject, a therapeutically effective amount of a composition comprising a first agent that inhibits hedgehog signaling pathway and a second agent that inhibits BCR-ABL, or pharmaceutically acceptable salts or pharmaceutical compositions thereof, thereby treating said BCR-ABL positive leukemia. For example, the compositions of the invention may be used to treat chronic myeloid leukemia or acute lymphocyte leukemia.

Furthermore, the present invention provides for the use of a therapeutically effective amount of a composition comprising a first agent that inhibits hedgehog signaling pathway and a second agent that inhibits BCR-ABL, or pharmaceutically acceptable salts or pharmaceutical compositions thereof, in the manufacture of a medicament for treating a cell proliferative disorder, particularly BCR-ABL positive leukemia, as defined in claim 5 and its dependent claims, all of which are incorporated here by reference.

In the above compositions and methods for using the compositions of the invention, the first agent in the inventive composition may bind to Smo. The first agent is cyclopamine or forskolin. The second agent in the inventive composition or combination is selected from the group consisting of

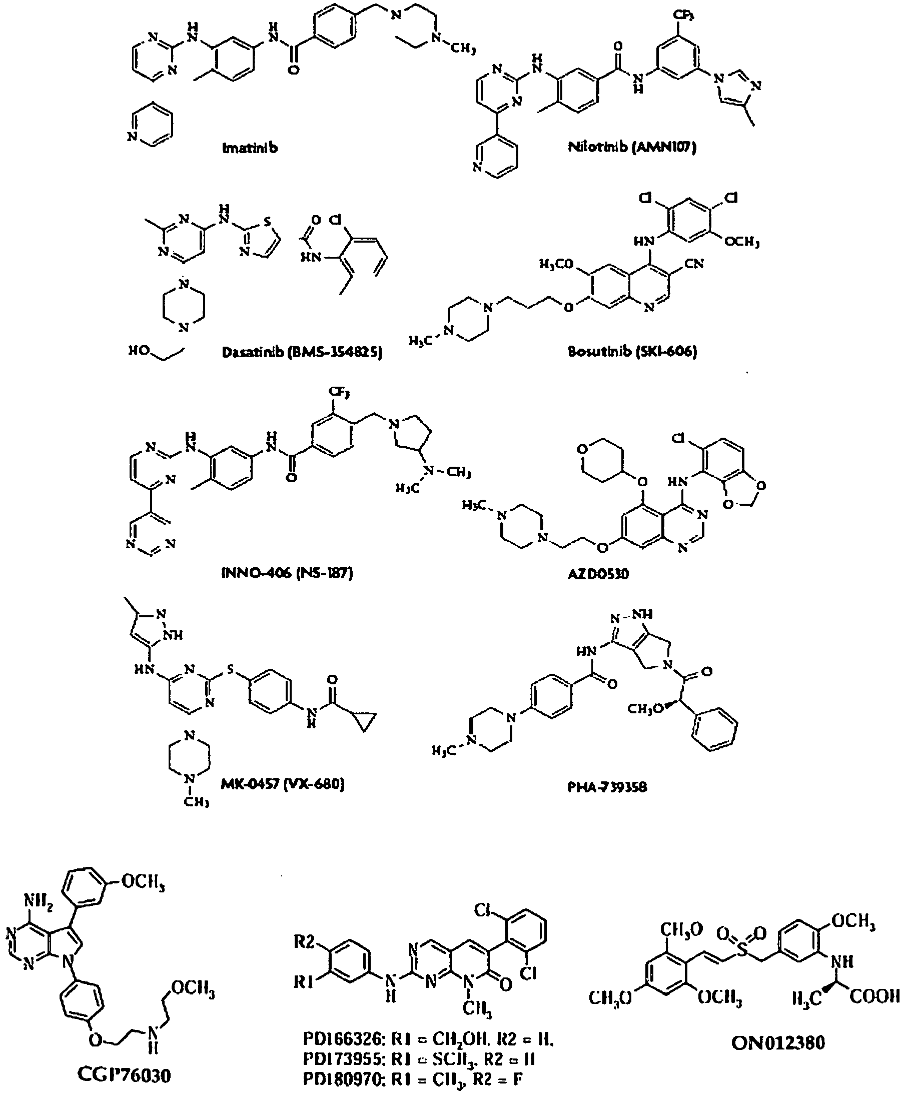

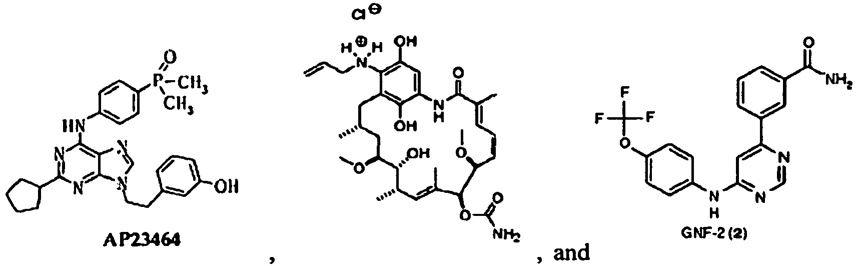

In the above compositions and combinations of the invention, and disclosed methods for using the compositions of the invention, the inventive composition may be administered to a system comprising cells or tissues. In some embodiments, the invention composition may be administered to a human or animal subject.

### Brief Description of the Drawings

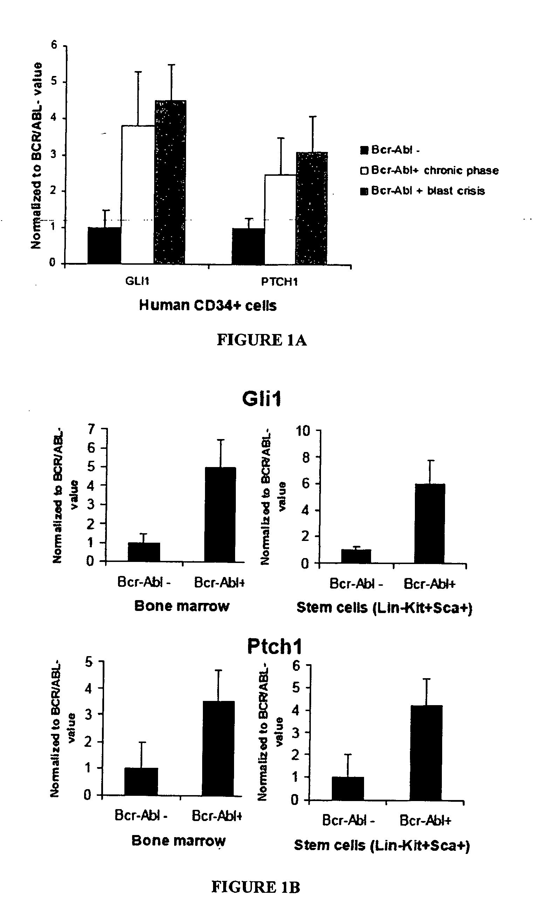

Figure 1A shows the transcript levels for Gli1 and Ptch1 in purified CD34+ cells from healthy patients and patients with CML in chronic phase or blast crisis (values normalized to CD34+ cells). Figure 1B shows the expression of Gli1 and Ptch1 transcript levels in BCR-ABL positive versus negative whole bone marrow or stem cells.

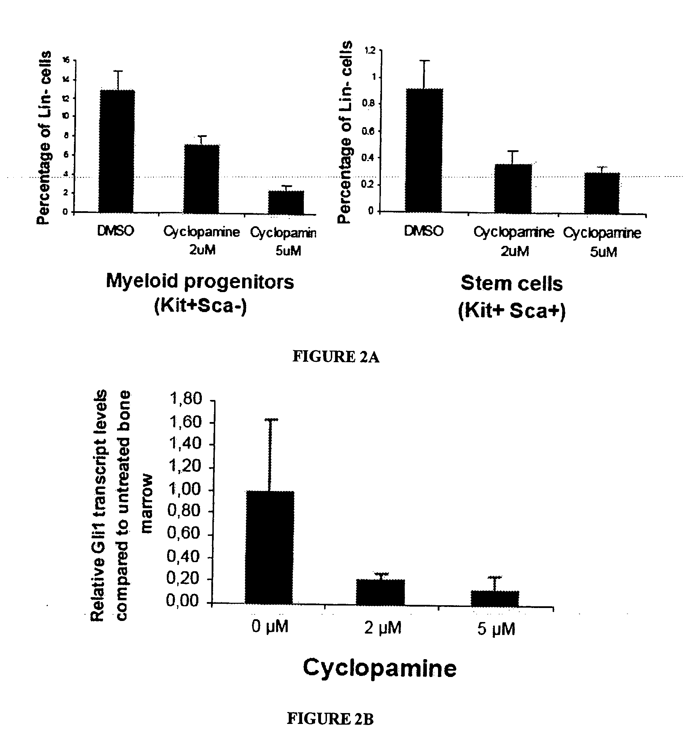

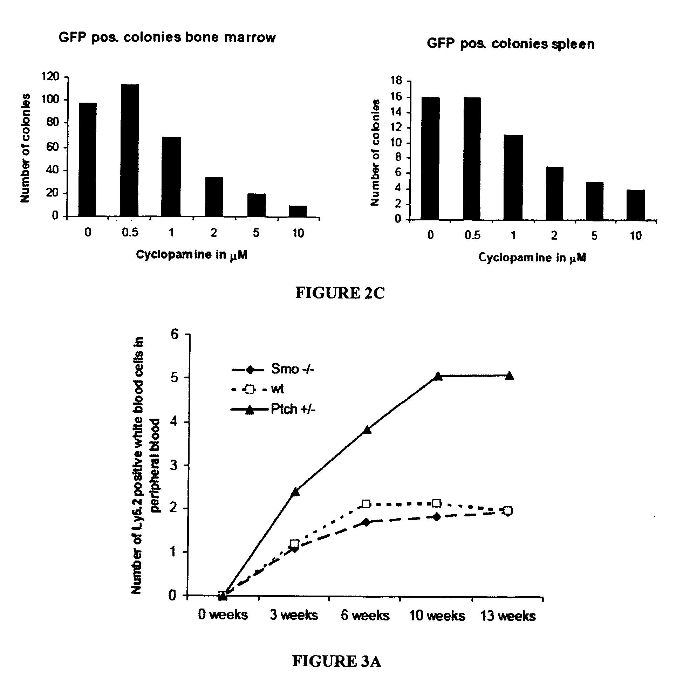

Figure 2A shows the percentage of BCR-ABL (GFP) positive myeloid progenitors (Lin-, Kit+, Sca-) and HSCs (Lin-, Kit+, Sca+) after treatment of mixed bone marrow cultures with cyclopamine for 72 hours. Figure 2B shows Gli1 expression after treatment of bone marrow of leukemic mice with cyclopamine. Figure 2C shows the total number of colonies counted 10 days after plating of cyclopamine treated mixed bone marrow cultures.

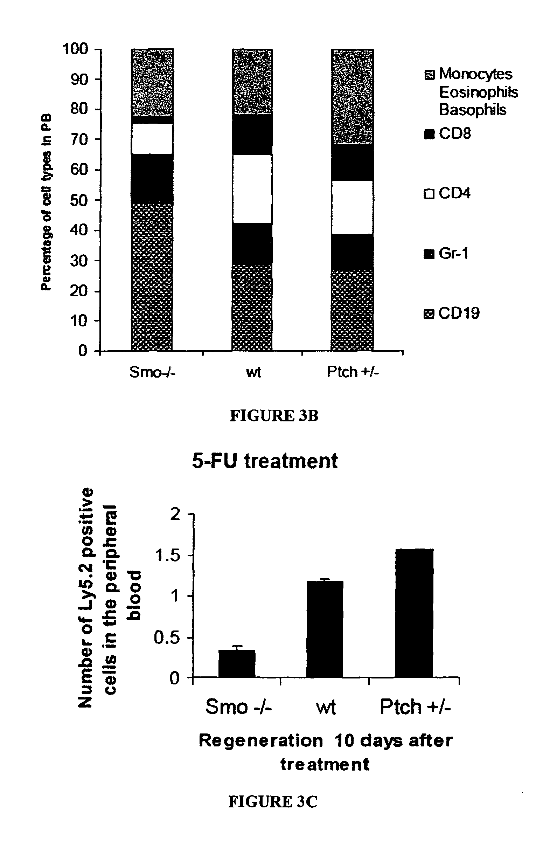

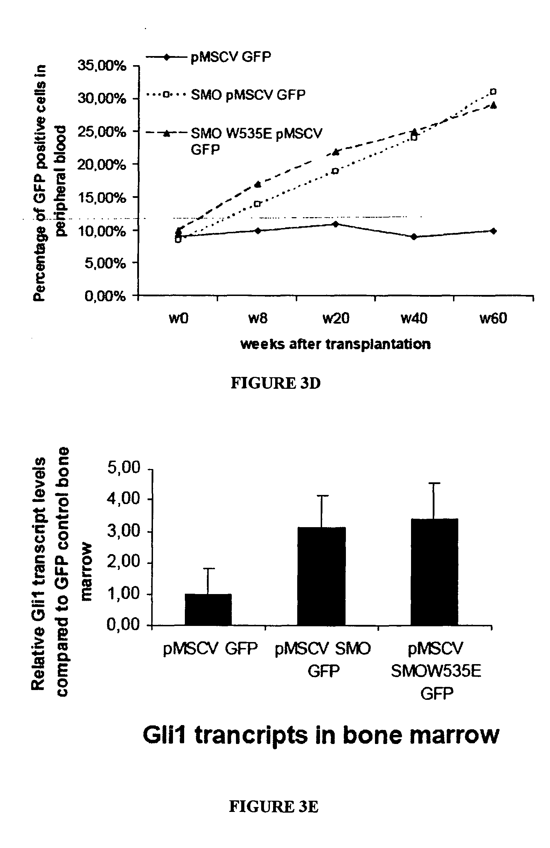

Figure 3A shows the number of Ly5.2 (embryos) positive cells in the peripheral blood after transplantation into PepC-Ly5.1 mice. Figure 3B shows the cell type distribution in Ly5.2 positive cells 10 weeks after transplantation in the peripheral blood. Figure 3C shows the regeneration of Ly5.2 positive cells in the peripheral blood after 5-FU treatment (150 mg/kg). Figure 3D shows the percentage of GFP positive cells in the peripheral blood of mice transplanted with bone marrow containing 10% GFP positive cells, 10% Smo GFP positive cells or 10% SMOW535E GFP positive cells over a period of 60 weeks. Figure 3E shows relative GLI1 transcript levels of bone marrow either infected with a pMSCV control vector or Smo GFP or SMOW535E GFP vector.

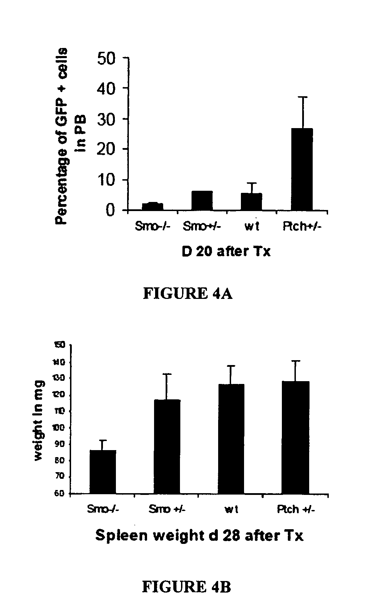

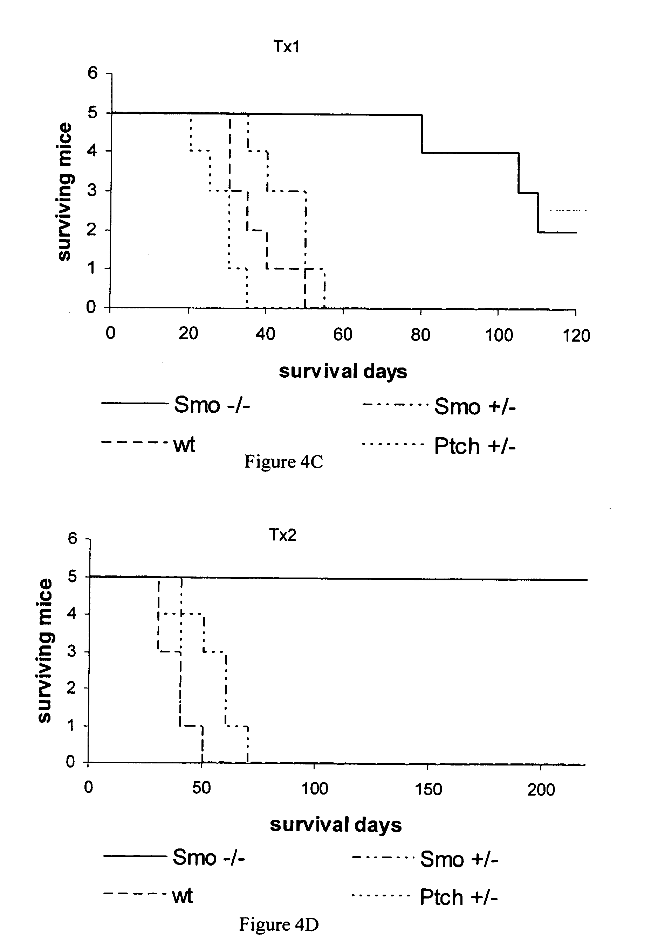

Figure 4A shows the number of BCR-ABL positive cells in the peripheral blood of transplanted mice 20 days after transplantation (Tx). Figure 4B shows the spleen weight of transplanted mice 28 days after Tx. Figure 4C shows the survival of mice transplanted with BCR-ABL infected fetal liver cells. Figure 4D shows the survival of mice retransplanted with 2*10E5 BCR-ABL (GFP) positive bone marrow cells.

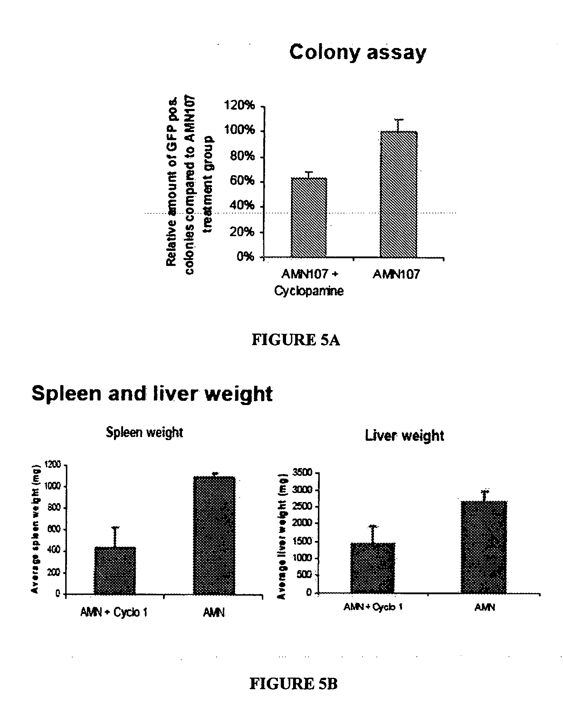

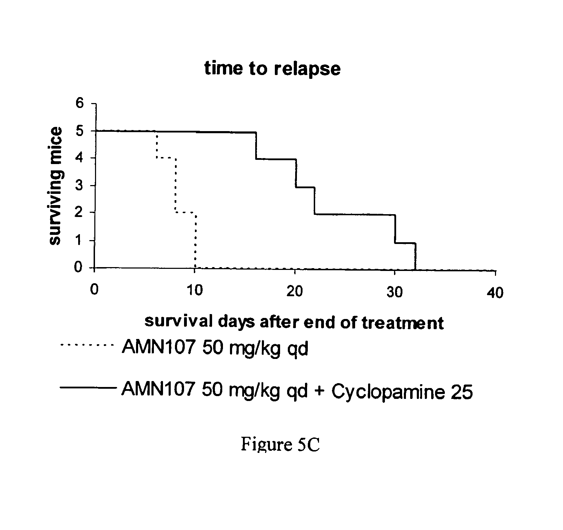

Figure 5A shows the relative amount of GFP positive bone marrow colonies of one femur in BCR-ABL+ mice treated with either AMN107 or a combination of AMN107 and Cyclopamine. Figure 5B shows the spleen and liver weight 8 days after end of treatment. Figure 5C shows the survival days after end of treatment with either AMN107 alone or the combination of AMN107 with cyclopamine.

### Definitions

Unless defined otherwise, all technical and scientific terms used herein have the same meaning as commonly understood by those of ordinary skill in the art to which this invention pertains. The following references provide one of skill with a general definition of many of the terms used in this invention: Oxford Dictionary of Biochemistry and Molecular Biology, Smith et al. (eds.), Oxford University Press (revised ed., 2000); Dictionary of Microbiology and Molecular Biology, Singleton et al. (eds.), John Wiley & Sons (3rd ed., 2002); and A Dictionary of Biology (Oxford Paperback Reference), Martin and Hine (Eds.), Oxford University Press (4th ed., 2000). In addition, the following definitions are provided to assist the reader in the practice of the invention.

The term "agent" or "test agent" includes any substance, molecule, element, compound, entity, or a combination thereof. It includes, but is not limited to, e.g., protein, polypeptide, small organic molecule, polysaccharide, polynucleotide, and the like. It can be a natural product, a synthetic compound, a chemical compound, or a combination of two or more substances. Unless otherwise specified, the terms "agent", "substance", and "compound" can be used interchangeably.

The term "analog" is used herein to refer to a molecule that structurally resembles a reference molecule but which has been modified in a targeted and controlled manner, by replacing a specific substituent of the reference molecule with an alternate substituent. Compared to the reference molecule, one skilled in the art would expect an analog to exhibit the same, similar, or improved utility. Synthesis and screening of analogs to identify variants of known compounds having improved traits (such as higher binding affinity for a target molecule) is an approach that is well known in pharmaceutical chemistry.

As used herein, "contacting" has its normal meaning and refers to combining two or more molecules (e.g., a small molecule organic compound and a polypeptide) or combining molecules and cells (e.g., a compound and a cell). Contacting can occur in vitro, e.g., combining two or more agents or combining a compound and a cell or a cell lysate in a test tube or other container. Contacting can also occur in a cell or in situ, e.g., contacting two polypeptides in a cell by coexpression in the cell of recombinant polynucleotides encoding the two polypeptides, or in a cell lysate.

The term "hedgehog" is used to refer generically to any member of the hedgehog family, including sonic, indian, desert and tiggy winkle. The term may be used to indicate protein or gene. The term is also used to describe homolog/ortholog sequences in different animal species.

The terms "hedgehog (Hh) signaling pathway" and "hedgehog (Hh) signaling" are used interchangeably and refer to the chain of events normally mediated by various members of the signaling cascade such as hedgehog, patched (Ptch), smoothened (Smo), and Gli. The hedgehog pathway can be activated even in the absence of a hedgehog protein by activating a downstream component. For example, overexpression of Smo will activate the pathway in the absence of hedgehog.

Hh signaling components or members of Hh signaling pathway refer to gene products that participate in the Hh signaling pathway. An Hh signaling component frequently affects the transmission of the Hh signal in cells/tissues, typically resulting in changes in degree of downstream gene expression level and/or phenotypic changes. Hh signaling components, depending on their biological function and effects on the final outcome of the downstream gene activation/expression, may be divided into positive and negative regulators. A positive regulator is an Hh signaling component that positively affects the transmission of the Hh signal, i.e., stimulates downstream biological events when Hh is present. Examples include hedgehog, Smo, and Gli. A negative regulator is an Hh signaling component that negatively affects the transmission of the Hh signal, i.e., inhibits downstream biological events when Hh is present. Examples include (but are not limited to) Ptch and SuFu.

Hedgehog signaling antagonists, antagonists of Hh signaling or inhibitors of Hh signaling pathway refer to agents that inhibit the bioactivity of a positive Hh signaling component (such as hedgehog, Ptch, or Gli) or down-regulate the expression of the Hh signaling component. They also include agents which up-regulate a negative regulator of Hh signaling component. A hedgehog signaling antagonist may be directed to a protein encoded by any of the genes in the hedgehog pathway, including (but not limited to) sonic, indian or desert hedgehog, smoothened, ptch-1, ptch-2, gli-1, gli-2, gli-3, etc.

A "heterologous sequence" or a "heterologous nucleic acid," as used herein, is one that originates from a source foreign to the particular host cell, or, if from the same source, is modified from its original form. Thus, a heterologous gene in a host cell includes a gene that, although being endogenous to the particular host cell, has been modified. Modification of the heterologous sequence can occur, e.g., by treating the DNA with a restriction enzyme to generate a DNA fragment that is capable of being operably linked to the promoter. Techniques such as site-directed mutagenesis are also useful for modifying a heterologous nucleic acid.

The term "homologous" when referring to proteins and/or protein sequences indicates that they are derived, naturally or artificially, from a common ancestral protein or protein sequence. Similarly, nucleic acids and/or nucleic acid sequences are homologous when they are derived, naturally or artificially, from a common ancestral nucleic acid or nucleic acid sequence. Homology is generally inferred from sequence similarity between two or more nucleic acids or proteins (or sequences thereof). The precise percentage of similarity between sequences that is useful in establishing homology varies with the nucleic acid and protein at issue, but as little as 25% sequence similarity is routinely used to establish homology. Higher levels of sequence similarity, e.g., 30%, 40%; 50%, 60%, 70%, 80%, 90%, 95% or 99% or more can also be used to establish homology.

A "host cell" refers to a prokaryotic or eukaryotic cell into which a heterologous polynucleotide can be introduced. The polynucleotide can be introduced into the cell by any means, e.g., electroporation, calcium phosphate precipitation, microinjection, transformation, viral infection, and/or the like.

The term "inhibiting" or "inhibition," in the context of tumor growth or tumor cell growth, refers to delayed appearance of primary or secondary tumors, slowed development of primary or secondary tumors, decreased occurrence of primary or secondary tumors, slowed or decreased severity of secondary effects of disease, or arrested tumor growth and regression of tumors. The term "prevent" or "prevention" refers to a complete inhibition of development of primary or secondary tumors or any secondary effects of disease. In the context of modulation of enzymatic activities, inhibition relates to reversible suppression or reduction of an enzymatic activity including competitive, uncompetitive, and noncompetitive inhibition. This can be experimentally distinguished by the effects of the inhibitor on the reaction kinetics of the enzyme, which may be analyzed in terms of the basic Michaelis-Menten rate equation. Competitive inhibition occurs when the inhibitor can combine with the free enzyme in such a way that it competes with the normal substrate for binding at the active site. A competitive inhibitor reacts reversibly with the enzyme to form an enzyme-inhibitor complex [EI], analogous to the enzyme-substrate complex.

The term "sequence identity" in the context of two nucleic acid sequences or amino acid sequences refers to the residues in the two sequences which are the same when aligned for maximum correspondence over a specified comparison window. A "comparison window" refers to a segment of at least about 20 contiguous positions, usually about 50 to about 200, more usually about 100 to about 150 in which a sequence may be compared to a reference sequence of the same number of contiguous positions after the two sequences are aligned optimally. Methods of alignment of sequences for comparison are well-known in the art. Optimal alignment of sequences for comparison may be conducted by the local homology algorithm of Smith and Waterman, Adv. Appl. Math. 1981, 2:482; by the alignment algorithm of Needleman and Wunsch, J. Mol. Biol. 1970, 48:443; by the search for similarity method of Pearson and Lipman, Proc. Nat. Acad. Sci U.S.A. 1988, 85:2444; or by computerized implementations of these algorithms (including, but not limited to CLUSTAL in the PC/Gene program by Intelligentics, Mountain View, CA; and GAP, BESTFIT, BLAST, FASTA, or TFASTA in the Wisconsin Genetics Software Package, Genetics Computer Group (GCG), 575 Science Dr., Madison, Wis., U.S.A.). The CLUSTAL program is well described by Higgins and Sharp, Gene 1988, 73:237-244; Higgins and Sharp, CABIOS 1989, 5:151-153; Corpet et al., Nucleic Acids Res. 1988, 16:10881-10890; Huang et al, Computer Applications in the Biosciences 1992, 8:155-165; and Pearson et al., Methods in Molecular Biology 1994, 24:307-331. Alignment is also often performed by inspection and manual alignment. In one class of embodiments, the polypeptides are at least 70%, generally at least 75%, optionally at least 80%, 85%, 90%, 95% or 99% or more identical to a reference polypeptide (e.g., a hedgehog molecule, e.g., as measured by BLASTP or CLUSTAL, or any other available alignment software using default parameters). Similarly, nucleic acids can also be described with reference to a starting nucleic acid, e.g., they can be 50%, 60%, 70%, 75%, 80%, 85%, 90%, 95%, 99% or more identical to a reference nucleic acid (e.g., as measured by BLASTN or CLUSTAL, or any other available alignment software using default parameters).

A "substantially identical" nucleic acid or amino acid sequence refers to a nucleic acid or amino acid sequence which comprises a sequence that has at least 90% sequence identity to a reference sequence using the programs described above (preferably BLAST) using standard parameters. The sequence identity is may be at least 95%, more particularly at least 98%, and in some examples, are at least 99%. For example, the BLASTN program (for nucleotide sequences) uses as defaults a word length (W) of 11, an expectation (E) of 10, M=5, N=-4, and a comparison of both strands. For amino acid sequences, the BLASTP program uses as defaults a word length (W) of 3, an expectation (E) of 10, and the BLOSUM62 scoring matrix (see Henikoff & Henikoff, Proc. Natl. Acad. Sci. USA 1989, 89:10915). Percentage of sequence identity is determined by comparing two optimally aligned sequences over a comparison window, wherein the portion of the polynucleotide sequence in the comparison window may comprise additions or deletions (i.e., gaps) as compared to the reference sequence (which does not comprise additions or deletions) for optimal alignment of the two sequences. The percentage is calculated by determining the number of positions at which the identical nucleic acid base or amino acid residue occurs in both sequences to yield the number of matched positions, dividing the number of matched positions by the total number of positions in the window of comparison and multiplying the result by 100 to yield the percentage of sequence identity. The substantial identity may exist over a region of the sequences that is at least about 50 residues in length, more particularly over a region of at least about 100 residues. In some examples, the sequences are substantially identical over at least about 150 residues, or the sequences may be substantially identical over the entire length of the coding regions.

The term "modulate" with respect to a biological activity of a reference protein (e.g., a hedgehog pathway member) or its fragment refers to a change in the expression level or other biological activities of the protein. For example, modulation may cause an increase or a decrease in expression level of the reference protein, enzymatic modification (e.g., phosphorylation) of the protein, binding characteristics (e.g., binding to another molecule), or any other biological (e.g., enzymatic), functional, or immunological properties of the reference protein. The change in activity can arise from, for example, an increase or decrease in expression of one or more genes that encode the reference protein, the stability of an mRNA that encodes the protein, translation efficiency, or from a change in other biological activities of the reference protein. The change can also be due to the activity of another molecule that modulates the reference protein (e.g., a kinase which phosphorylates the reference protein).

Modulation of a reference protein can be up-regulation (i.e., activation or stimulation) or down-regulation (i.e. inhibition or suppression). The mode of action of a modulator of the reference protein can be direct, e.g., through binding to the protein or to genes encoding the protein, or indirect, e.g., through binding to and/or modifying (e.g., enzymatically) another molecule which otherwise modulates the reference protein.

The term "subject" includes mammals, especially humans. It also encompasses other non-human animals such as cows, horses, sheep, pigs, cats, dogs, mice, rats, rabbits, guinea pigs, monkeys.

The term "treat" or "treatment" refers to arrested tumor growth, and to partial or complete regression of tumors. The term "treating" includes the administration of compounds or agents to prevent or delay the onset of the symptoms, complications, or biochemical indicia of a disease (e.g., leukemia), alleviating the symptoms or arresting or inhibiting further development of the disease, condition, or disorder. Treatment may be prophylactic (to prevent or delay the onset of the disease, or to prevent the manifestation of clinical or subclinical symptoms thereof) or therapeutic suppression or alleviation of symptoms after the manifestation of the disease.

A "variant" of a reference molecule refers to a molecule substantially similar in structure and biological activity to either the entire reference molecule, or to a fragment thereof. Thus, provided that two molecules possess a similar activity, they are considered variants as that term is used herein even if the composition or secondary, tertiary, or quaternary structure of one of the molecules is not identical to that found in the other, or if the sequence of amino acid residues is not identical.

### Modes of Carrying Out the Invention

The invention provides compositions and pharmaceutical compositions thereof, which may be useful for inhibiting tumor cell growth and for treating a variety of cancers.

More particularly, the invention provides a composition comprising a first agent that inhibits hedgehog signaling pathway and a second agent that inhibits BCR-ABL, each as defined above. The composition may be used for inhibiting the growth and proliferation of hematopoietic tumors of lymphoid and myeloid, and for treating cancers known to be associated with protein tyrosine kinases such as, for example, Src, BCR-ABL and c-kit. In particular embodiments, the composition may be used for treating BCR-ABL-positive chronic myeloid leukemia (CML) and acute lymphocytic leukemia (ALL).

Chronic myeloid leukemia is characterized by the expansion of a leukemic stem cell clone carrying a Philadelphia translocation, which outgrows the non-malignant hematopoietic stem cells. The present invention is predicated in part, on the discovery that BCR-ABL directly enhances self renewal of hematopoietic stem and progenitor cells by activating the hedgehog signaling pathway through upregulation of Smo. BCR-ABL upregulates Smo expression and activates the hedgehog signaling pathway in mouse and human HSCs.

Pharmacological inhibition of Smo activity in BCR-ABL positive bone marrow cultures inhibits colony forming capacity of BCR-ABL positive self renewing cells *in vitro.* Combined treatment of leukemic mice with AMN107 (Abl inhibitor) and cyclopamine (Smo inhibitor) led to a reduction of BCR-ABL positive self-renewing cells *in vivo* and enhanced the time to relapse more than 3-fold compared to mice treated with AMN107 alone. Thus, BCR-ABL enhances the self renewal of leukemic stem cells through intrinsic activation of hedgehog signaling by upregulation of Smo. Therefore Hh pathway inhibition alone or in combination with Abl inhibitors could serve as an effective therapeutic strategy to reduce the malignant stem cell pool in BCR-ABL positive leukemias.

The therapeutic methods employ an agent that inhibits the hedgehog signaling pathway in combination with an agent that inhibits BCR-ABL, for inhibiting the growth and proliferation of cancer cells, particularly cancers of the blood and lymphatic systems, such as leukemia and myelomas. These methods involve contacting such a tumor cell (in vitro or in vivo) with a composition comprising an inhibitor of the Hh signaling pathway and an inhibitor of BCR-ABL.

### A. Agents that Inhibit Hedgehog Signaling

Various agents that inhibit the hedgehog signaling pathway known in the art may be used to practice the disclosed matter or the invention embodiments. These include organic compounds that directly or indirectly modulate a biological activity (e.g., enzymatic activity) of a member of the hedgehog signaling pathway. They also include agents that specifically target a gene or an mRNA which encode a member of the hedgehog signaling pathway. Other antagonists of hedgehog signaling pathway ma also be employed to practice the methods, including antibodies or other binding agents which target a member of the hedgehog signaling pathway (e.g., a transmembrane receptor).

The Hh signaling pathway is a developmental pathway shown to play a role in fetal and adult hematopoietic stem cells (HSCs). (Trowbridge et al., Proc Natl Acad Sci USA 2006, 103:14134-9). Hedgehog ligands (Shh, Ihh and Dhh) produced by stroma cells bind to the seven-transmembrane receptor Ptch. Ligand binding to Ptch releases Ptch binding to Smo, a second seven-transmembrane receptor. This results in a conformational change of Smo and following activation of the downstream signaling pathway with induction of the Gli transcription factors (Gli1 Gli2, Gli3) and transcription of target genes like Gli1, Ptch1, cyclin D1 and Bcl2 (Duman-Scheel et al., Nature 2002, 417:299-304). During early embryogenesis, secretion of Indian hedgehog by visceral endoderm induces formation of primitive hematopoietic cells in the yolk sac of murine embryos. Zebrafish embryos with defective mutations in Smo or treated with the Hh signaling inhibitor cyclopamine display defects in adult HSC formation (Gering et al., Dev. Cell. 2005, 8:389-400). Recent publications indicate a role of hedgehog signaling in cell cycle regulation of adult HSCs (Trowbridge et al., supra).

To practice the therapeutic methods of the disclosure, a number of Hh signaling pathway components may be modulated. These include positive regulators of Hh signaling which may be antagonized and negative regulators of Hh signaling which may be agonized. Hedgehog (Hh) (including, e.g., Ihh, Shh, and Dhh), Smoothened (Smo), and Gli are examples of positive regulators, while Patched (Ptch) and Suppressor of Fused (Fu) are negative regulators. All Hh signaling pathway genes in various species may be easily cloned based on sequences readily available from public and proprietary databases, such as GenBank, EMBL, or FlyBase.

Many inhibitors of the hedgehog signaling pathway are known in the art and may be readily employed in the practice of the hedgehog signaling pathway. Some Hh signaling antagonists are small molecule compounds which target a key member of Hh pathway such as Smo, e.g., cyclopamine, SANT1 and Cur61414 (Katoh et al., Maycer Biol Ther.2005, 4:1050-4; and Williams et al., Proc Natl Acad Sci USA. 2003, 100:4616-21). For example, in an invention embodiment cyclopamine inhibits hedgehog signaling pathway by directly binding to Smo. Other antagonists of Hh signaling indirectly inhibit Hh pathway by acting on another molecule which in turn affects Hh signaling. For example, in another invention embodiment forskolin activates protein kinase A which in turn blocks Hh signaling downstream of Smo (See, e.g., Yao et al., Dev Biol. 2002, 246:356-65). Additional organic compound inhibitors of Hh signaling have been described in, e.g., US patent applications US20060063779 (Gunzner et al., 2006), US20050222087 (Beachy, 2005) and US 20010034337 (Dudek et al., 2001). Any of these Hh signaling antagonists may be employed to carry out the therapeutic methods of the present disclosure. Some of the compounds may be obtained commercially (e.g., cyclopamine or SANT-1). Others may be easily synthesized using methods routinely practiced in the art of organic chemistry.

In some disclosure embodiments, the employed antagonist of Hh signaling is a binding agent which specifically inhibits activation of the Hh signaling pathway. For example, when not bound by its ligand, the transmembrane receptor Ptch binds to Smo and blocks its function. Thus, a binding agent which may inhibit or block hedgehog binding to Ptch may be used to antagonize Hh signaling. Antagonist antibodies or antibody homologs as well as other molecules such as soluble forms of the natural binding proteins for hedgehog are useful. For example, monoclonal antibodies such an anti-hedgehog or anti-patched antibody homolog may be used to practice the methods disclosed herein. These antibodies should be able to block hedgehog binding to Ptch but do not activate Hh signaling.

In some methods, an antibody that specifically binds to a hedgehog polypeptide may be used. Using neutralizing antibodies against hedgehog to inhibit Hh signaling is well known and routinely practiced in the art. See, e.g., Ahlgren et al., Curr Biol. 1999, 9:1304-14; Cobourne et al., J Dent Res. 2001, 80:1974-9; Hall et al., Dev Biol. 2003, 255:263-77; and Berman et al., Nature 2003, 425:846-51. An example of such hedgehog neutralizing antibodies is monoclonal antibody clone 5E1. This antibody may be obtained from Developmental Studies Hybridoma Bank, University of Iowa.

In some other disclosure embodiments, soluble forms of binding agents derived from Ptch may be used. These include soluble Ptch peptides, Ptch fusion proteins, or bifunctional Ptch/Ig fusion proteins. Some of these soluble agents contain a polypeptide fragment with a sequence identical or substantially identical to that of a Ptch fragment that harbors its ligand binding site. For example, a soluble form of Ptch or a fragment thereof which binds to hedgehog may be employed to compete with Ptch on cells for binding to hedgehog, thereby blocking activation of Hh signaling. In addition, soluble hedgehog mutants that bind Ptch but do not elicit hedgehog-dependent signaling may also be used in the practice of the disclosure.

Some therapeutic applications directed to human subjects employ antibody antagonists of Hh pathway that are of human origin. These include human antibodies, humanized antibodies, chimeric antibodies, Fab, Fab', F(ab')2 or F(v) antibody fragments, as well as monomers or dimers of antibody heavy or light chains or mixtures thereof. A chimeric antibody is an antibody homolog in which all or part of the hinge and constant regions of an immunoglobulin light chain, heavy chain, or both, have been substituted with the corresponding regions from a human immunoglobulin light chain or heavy chain. A humanized antibody is an antibody homolog which, in addition to having human constant region sequences, also has some or all of its non-CDR amino acid residues in the variable regions being replaced with corresponding amino acids from a human immunoglobulin. Human antibodies are antibody homologs in which all of the amino acids of an immunoglobulin light and heavy chain are derived from a human source.

Antibody homologs include intact antibodies consisting of immunoglobulin light and heavy chains linked via disulfide bonds. It also encompasses a protein comprising one or more polypeptides selected from immunoglobulin light chains, immunoglobulin heavy chains and antigen-binding fragments thereof which are capable of binding to one or more antigens (i.e., hedgehog or patched). The component polypeptides of an antibody homolog composed of more than one polypeptide may optionally be disulfide-bound or otherwise covalently crosslinked. Antibody homologs also include portions of intact antibodies that retain antigen-binding specificity, for example, Fab fragments, Fab' fragments, F(ab')2 fragments, F(v) fragments, heavy chain monomers or dimers, light chain monomers or dimers, dimers consisting of one heavy and one light chain, and the like. Thus, antigen-binding fragments, as well as full-length dimeric or trimeric polypeptides derived from the above-described antibodies are also useful in the practice of the present disclosure.

Anti-hedgehog and anti-Patched antibody homologs may be produced using methods well known in the art, e.g., Monoclonal Antibodies--Production, Engineering And Clinical Applications, Ritter et al., Eds., Cambridge University Press, Cambridge, UK, 1995; and Harlow and Lane, Antibodies, A Laboratory Manual, Cold Spring Harbor Press, 3rd ed., 2000. Human monoclonal antibody homologs against hedgehog or patched may be prepared using in vitro-primed human splenocytes, as described by Boemer et al., J. Immunol. 1991, 147:86-95. Alternatively, they may be prepared by methods described in, e.g., Persson et al., Proc. Nat. Acad. Sci. USA 1991, 88: 2432-2436; Huang and Stollar, J. Immunol. Methods 1991, 141: 227-236; U.S. Patent Application Ser. No. 10/778,726 (Publication No. 20050008625); and U.S. Pat. Nos. 5,798,230 and 5,789,650. Humanized recombinant antibody homolog having the capability of binding to a hedgehog or patched protein may be generated using methods described in, e.g., Riechmann et al., Nature 1988, 332: 323-327; Verhoeyen et al., Science1988, 239: 1534-1536; Queen et al., Proc. Nat. Acad. Sci. USA 1989, 86:10029; and Orlandi et al., Proc. Natl. Acad. Sci. USA 1989, 86:3833.

Some therapeutic methods of the present disclosure employ nucleic acid agents that antagonize the hedgehog signaling pathway. Typically, these agents down-regulate expression of one or more genes encoding positive Hh signaling components such as hedgehog, Smo or Gli. These include double-stranded RNAs such as short interfering RNA (siRNA) and short hairpin RNA (shRNAs), microRNA (miRNA), anti-sense nucleic acid, and complementary DNA (cDNA). Interference with the function and expression of endogenous genes by double-stranded RNAs has been shown in various organisms such as C. elegans as described, e.g., in Fire et al., Nature 1998, 391:806-811; drosophilia as described, e.g., in Kennerdell et al., Cell 1998, 95:1017-1026; and mouse embryos as described, e.g., in Wianni et al., Nat. Cell Biol. 2000, 2:70-75. Such double-stranded RNA may be synthesized by in vitro transcription of single-stranded RNA read from both directions of a template and in vitro annealing of sense and antisense RNA strands. Double-stranded RNA may also be synthesized from a cDNA vector construct in which a target gene is cloned in opposing orientations separated by an inverted repeat. Following cell transfection, the RNA is transcribed and the complementary strands reannealed. To antagonize Hh signaling in the present disclosure, double-stranded RNA targeting a positive regulator of Hh signaling pathway may be introduced into a cell (e.g., a lymphoma cell) by transfection of an appropriate construct.

In some embodiments, siRNAs antagonists of Hh signaling may be employed in the practice of the disclosure. The siRNA antagonists may modulate hedgehog signaling at any point in the hedgehog signaling pathway. For example, they may regulate Hh signaling by antagonizing hedgehog itself, or any other positive Hh signaling components such as Smo or Gli. SiRNAs are typically around 19-30 nucleotides in length, and preferably 21-23 nucleotides in length. They are double stranded, and may include short overhangs at each end. SiRNAs may be chemically synthesized or recombinantly produced using methods known in the art. Recombinant production of siRNAs in general involves transcription of short hairpin RNAs (shRNAs) that are efficiently processed to form siRNAs within cells. See, e.g., Paddison et al. Proc Natl Acad Sci USA 2002, 99:1443-1448; Paddison et al. Genes & Dev. 2002, 16:948-958; Sui et al. Proc Natl Acad Sci USA 2002, 8:5515-5520; Brummelkamp et al. Science 2002, 296:550-553; Caplen et al., Proc Natl Acad Sci USA 2001, 98:9742-9747; and Elbashir et al., EMBO J. 2001, 20:6877-88.

In some disclosure embodiments, the nucleic acid antagonists of Hh signaling may be double stranded hairpin RNA. The hairpin RNAs may be synthesized exogenously or may be formed by transcribing from RNA polymerase III promoters in vivo. Examples of making and using such hairpin RNAs for gene silencing in mammalian cells are described in, for example, Paddison et al., Genes Dev. 2002, 16:948-58; McCaffrey et al., Nature 2002, 418:38-9; McManus et al., RNA 2002, 8:842-50; and Yu et al., Proc Natl Acad Sci USA 2002, 99:6047-52. Preferably, such hairpin RNAs are engineered in cells or in an animal to ensure continuous and stable suppression of a desired gene. It is known in the art that siRNAs may be produced by processing a hairpin RNA in the cell.

### B. Agents that Inhibit BCR-ABL

Various BCR-ABL inhibitors known in the art may be used to practice the disclosure or where mentioned especially the invention, including but not limited to ABL inhibitors, inhibitors of both ABL and Src-family kinases, Aurora kinase inhibitors, and non-ATP competitive inhibitors of BCR-ABL.

The Src family of tyrosine kinases modulates multiple intracellular signal transduction pathways involved in cell growth, differentiation, migration and survival, many of which are involved in oncogenesis, tumor metastasis and angiogenesis. (Weisberg et al., Nat. Rev. Cancer 2007, 7:345-356). Many kinases from the Src family are expressed in hematopoietic cells (Blk, Fgr, Fyn, Hck, Lck, Lyn, c-Src and Yes). In addition, BCR-ABL has been shown to be capable of activating Src kinases both through phosphorylation and merely by binding Src proteins. Furthermore, cell lysates from imatinib-resistant patients have been found to over-express Lyn kinase, and the proliferation of human CML K562 cells selected for resistance to imatinib, which also over-express Lyn, is inhibited by the Abl/Src inhibitor, PD180970, in an invention embodiment. Since Src family kinases regulate downstream elements of the BCR-ABL signaling cascade, inhibition of these enzymes could therefore provide synergy with BCR-ABL inhibition, and potentially counteract the availability of alternative survival pathways which CML cells could utilize in the face of BCR-ABL inhibition. Therapy with combined BCR-ABL and Src-family kinase inhibitors might also therefore counteract the oncogenic potential of drug-resistant mutant forms of BCR-ABL in CML and/or ALL. (Manley et al., Biochim. Biophys. Acta 2005, 1754:3-13). Dasatinib (BMS-354825), bosutinib (SKI-606), INNO-404 (NS-187) and AZD05030 are examples of dual ABL-Src inhibitors useful in accordance with the invention embodiments.

The Aurora family of serine/threonine kinases is important for mitotic progression. Aurora-A has been reported to be overexpressed in various human cancers, and its overexpression induces aneuploidy, centrosome amplification and tumorigenic transformation in cultured human and rodent cells. (Zhang et al., Oncogene 2004, 23:8720-30). In the invention embodiments, MK-0457 (Merck; originally developed by Vertex Pharmaceuticals as VX-680), a potent inhibitor of all three Aurora kinases and FLT3 in the nanomolar range, is a moderate to strong inhibitor of ABL and JAK2, which are relevant targets for a range of myeloproliferative disorders. MK-0457 also inhibits the autophosphorylation ofT315I mutant BCR-ABL in transformed Ba/F3 cells with an IC₅₀ of ~5 µM, although it inhibits cell proliferation at submicromolar concentrations.

A potential alternative approach to ATP-competitive BCR-ABL inhibition is to use molecules that inhibit the kinase activity either by a non-ATP competitive allosteric mechanism or by preventing the binding of substrates to the kinase. This strategy has the advantage that the imatinib-resistant mutants are unlikely to be resistant to such inhibitors, owing to the different binding sites. High-throughput screening for inhibitors of BCR-ABL-dependent cell proliferation resulted in the identification of 3-[6-[[4-(trifluoromethoxy)phenyl]amino]-4-pyrimidinyl]benzamide (GNF-2) as a prototype inhibitor in the invention embodiments, which bound to the myristoyl binding site of BCR-ABL, resulting in the allosteric inhibition of ABL tyrosine kinase activity. GNF-2 inhibits the proliferation of Ba/F3 cells transfected with p210 non-mutated BCR-ABL, as well as with the E255V and M351T mutant forms of the enzyme. (Weisberg et al., Nat. Rev. Cancer 2007, supra).

Table 1 shows the BCR-ABL inhibitors which may be used to practice the invention, including nilotinib (AMN107), imatinib (STI571), 2,6,9-trisubstituted purine analog AP23464, AZD-0530, bosutinib, CPG070603, pyrido[2,3-d]pyrimidine compound dasatinib, PD 166326, PD173955, PD180970), ON012380, 3-substituted benzamide derivative Inn-406, MK-0457, PHA-739358 and GNF-2. (See e.g., Weisberg et al., Nat. Rev. Cancer 2007, supra; Tauchi et al., Int. J. Hematology 2006, 83:294-300; Manley et al., Biochim. Biophys. Acta 2005, supra; Ge et al., J. Med. Chem. 2006, 49:4606-4615; Adrian et al., Nat. Chem. Biol. 2006, 2:95-102; Asaki et al., Bioorg. Med. Chem. Lett. 2006, 16:1421-1425.

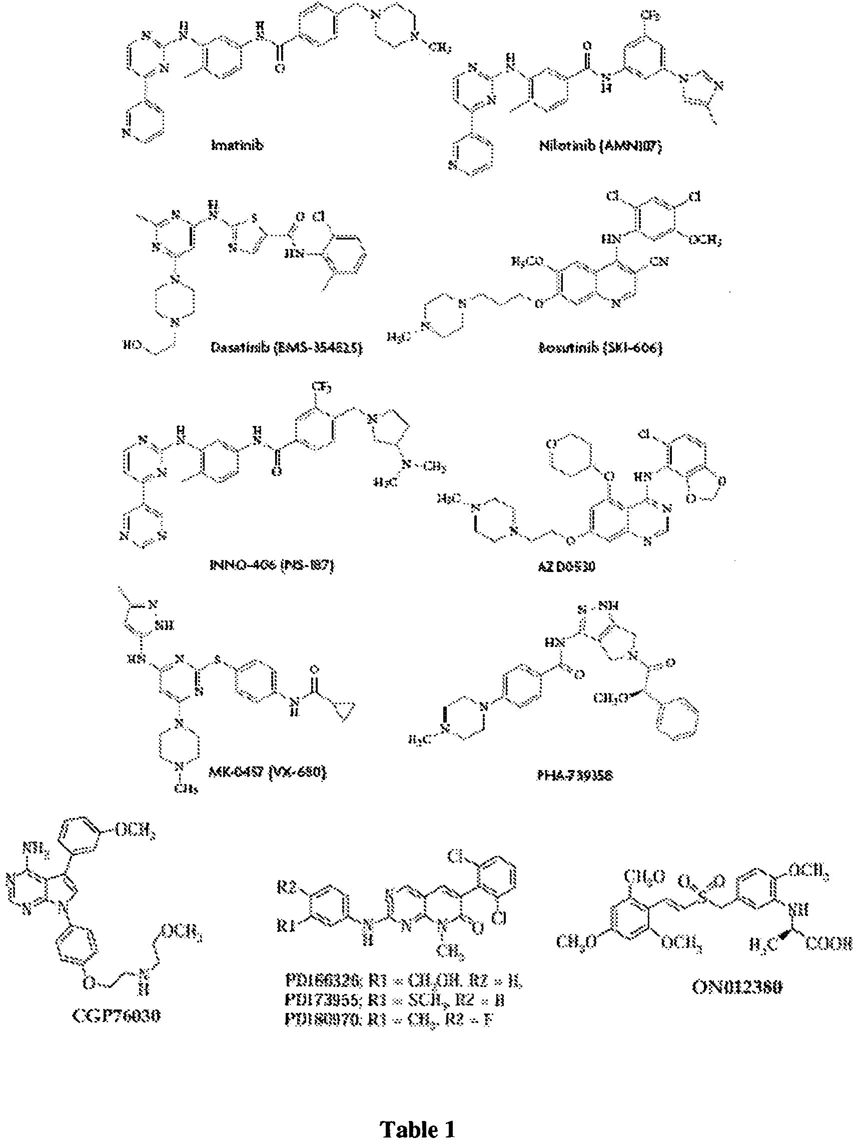

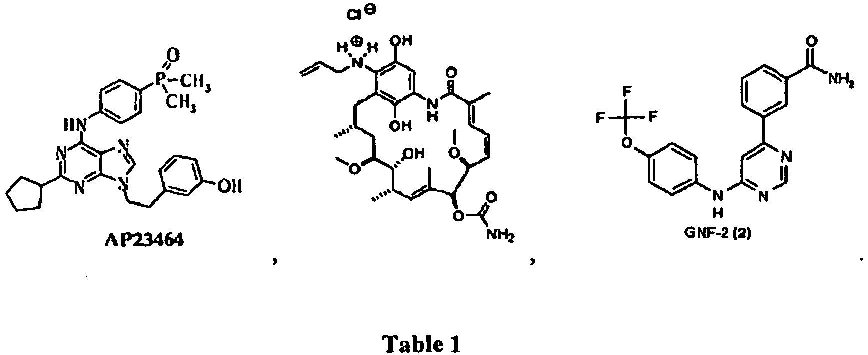

### C. Diseases and Conditions to be Treated

The combination of the present invention may be used for treating a variety of cancers. In one embodiment, the invention provides an agent that inhibits the hedgehog signaling pathway as defined above in combination with an agent that inhibits BCR-ABL as defined above, for inhibiting the growth and proliferation of hematopoietic tumors of lymphoid lineage including leukemia, acute lymphocytic leukemia (ALL), acute lymphoblastic leukemia, B-cell lymphoma, T-cell lymphoma, Hodgkins lymphoma, non-Hodgkins lymphoma, hairy cell lymphoma, histiocytic lymphoma, and Burkitts lymphoma; and hematopoietic tumors of myeloid lineage including acute and chronic myelogenous leukemias (CML), myelodysplastic syndrome, myeloid leukemia, and promyelocytic leukemia.

The combination of the present invention are also useful for treating cancers known to be associated with protein tyrosine kinases such as, for example, Src, BCR-ABL and c-kit. In particular embodiments, the combination of the present invention are useful for treating cancers that are sensitive to and resistant to chemotherapeutic agents that target BCR-ABL and c-kit. In particular embodiments, the combination of the present invention may be used for treating BCR-ABL-positive CML and ALL.

Chronic myelogenous leukemia (CML) is a cancer of the bone marrow characterized by increased and unregulated clonal proliferation of predominantly myeloid cells in the bone marrow. Its annual incidence is 1-2 per 100,000 people, affecting slightly more men than women. CML represents about 15-20% of all cases of adult leukemia in Western populations, about 4,500 new cases per year in the U.S. or in Europe. (Faderl et al., N. Engl. J. Med. 1999, 341: 164-72).

CML is a clonal disease that originates from a single transformer hematopoietic stem cell (HSC) or multipotent progenitor cell (MPP) harboring the Philadelphia translocation t(9/22). The expression of the gene product of this translocation, the fusion oncogene BCR-ABL, induces molecular changes which result in expansion of the malignant hematopoiesis including the leukemic stem cell (LSC) pool and the outgrowth and suppression of non-malignant hematopoiesis (Stam et al., Mol Cell Biol. 1987, 7:1955-60). Myeloid cells (granulocytes, monocytes, megakaryocytes, erythrocytes), but also B- and T-cells express BCR-ABL, indicating the MPP or HSC as the start point of the disease. (Fialkow et al., J. Clin. Invest. 1978, 62:815-23; Takahashi et al., Blood 1998, 92:4758-63). In contrast to oncogenes causing AML, like MOZ-TIF2 or MLL-ENL, BCR-ABL does not confer self-renewal properties to committed progenitor cells, but rather utilizes and enhances the self-renewal properties of existing self-renewing cells, like HSCs or MPPs. During the course of the disease, the leukemic stem cell pool expands and in the final stage, the blast crisis, nearly all CD34+CD38- cells carry the Philadelphia translocation.

Imatinib mesylate (STI571, GLEEVEC®) is becoming the standard of therapy for CML with response rates of more than 96 %, and works by inhibiting the activity of BCR-ABL. However, despite initial success, patients eventually develop resistance to imatinib mesylate due to acquisition of point mutations in BCR-ABL. In view of the limitations of imatinib mesylate, there is a need for improved methods for treating CML.

In addition, it is contemplated that the combination of the present invention may be used for treating carcinoma including that of the bladder (including accelerated and metastatic bladder cancer), breast, colon (including colorectal cancer), kidney, liver, lung (including small and non-small cell lung cancer and lung adenocarcinoma), ovary, prostate, testes, genitourinary tract, lymphatic system, rectum, larynx, pancreas (including exocrine pancreatic carcinoma), esophagus, stomach, gall bladder, cervix, thyroid, and skin (including squamous cell carcinoma); tumors of the central and peripheral nervous system including astrocytoma, neuroblastoma, glioma, and schwannomas; tumors of mesenchymal origin including fibrosarcoma, rhabdomyosarcoma, and osteosarcoma; and other tumors including melanoma, xeroderma pigmentosum, keratoacanthoma, seminoma, thyroid follicular cancer, and teratocarcinoma. It is also contemplated that the combinations of the present invention may be used for treating mastocytosis, germ cell tumors, pediatric sarcomas, and other cancers.

The therapeutic methods described herein may be used in combination with other cancer therapies. For example, Hh antagonists in combination with BCR-ABL inhibitors may be administered adjunctively with any of the treatment modalities, such as chemotherapy, radiation, and/or surgery. For example, they can be used in combination with one or more chemotherapeutic or immunotherapeutic agents; and may be used after other regimen(s) of treatment is concluded. Examples of chemotherapeutic agents which may be used in the compositions of the invention and for use in the methods of the invention include but are not limited to anthracyclines, alkylating agents (e.g., mitomycin C), alkyl sulfonates, aziridines, ethylenimines, methylmelamines, nitrogen mustards, nitrosoureas, antibiotics, antimetabolites, folic acid analogs (e.g., dihydrofolate reductase inhibitors such as methotrexate), purine analogs, pyrimidine analogs, enzymes, podophyllotoxins, platinum-containing agents, interferons, and interleukins.

Particular examples of known chemotherapeutic agents which may be used in the compositions and methods of the invention include, but are not limited to, busulfan, improsulfan, piposulfan, benzodepa, carboquone, meturedepa, uredepa, altretamine, triethylenemelamine, triethylenephosphoramide, triethylenethiophosphoramide, trimethylolomelamine, chlorambucil, chlomaphazine, cyclophosphamide, estramustine, ifosfamide, mechlorethamine, mechlorethamine oxide hydrochloride, melphalan, novembichin, phenesterine, prednimustine, trofosfamide, uracil mustard, carmustine, chlorozotocin, fotemustine, lomustine, nimustine, ranimustine, dacarbazine, mannomustine, mitobronitol, mitolactol, pipobroman, aclacinomycins, actinomycin F(1), anthramycin, azaserine, bleomycin, cactinomycin, carubicin, carzinophilin, chromomycin, dactinomycin, daunorubicin, daunomycin, 6-diazo-5-oxo-1-norleucine, doxorubicin, epirubicin, mitomycin C, mycophenolic acid, nogalamycin, olivomycin, peplomycin, plicamycin, porfiromycin, puromycin, streptonigrin, streptozocin, tubercidin, ubenimex, zinostatin, zorubicin, denopterin, methotrexate, pteropterin, trimetrexate, fludarabine, 6-mercaptopurine, thiamiprine, thioguanine, ancitabine, azacitidine, 6-azauridine, carmofur, cytarabine, dideoxyuridine, doxifluridine, enocitabine, floxuridine, fluorouracil, tegafur, L-asparaginase, pulmozyme, aceglatone, aldophosphamide glycoside, aminolevulinic acid, amsacrine, bestrabucil, bisantrene, carboplatin, cisplatin, defofamide, demecolcine, diaziquone, elfornithine, elliptinium acetate, etoglucid, etoposide, flutamide, gallium nitrate, hydroxyurea, interferon-alpha, interferon-beta, interferon-gamma, interleukin-2, lentinan, lonidamine, prednisone, dexamethasone, leucovorin, mitoguazone, mitoxantrone, mopidamol, nitracrine, pentostatin, phenamet, pirarubicin, podophyllinic acid, 2-ethylhydrazide, procarbazine, razoxane, sizofiran, spirogermanium, paclitaxel, tamoxifen, teniposide, tenuazonic acid, triaziquone, 2,2',2"-trichlorotriethylamine, urethane, vinblastine, vincristine, and vindesine.

The present methods may be used to treat primary, relapsed, transformed, or refractory forms of cancer. Often, patients with relapsed cancers have undergone one or more treatments including chemotherapy, radiation therapy, bone marrow transplants, hormone therapy, surgery, and the like. Of the patients who respond to such treatments, they may exhibit stable disease, a partial response (i.e., the tumor or a cancer marker level diminishes by at least 50%), or a complete response (i.e., the tumor as well as markers become undetectable). In either of these scenarios, the cancer may subsequently reappear, signifying a relapse of the cancer.

### D. Pharmaceutical Compositions and Administration

The compositions of the present invention may be administered alone under sterile conditions to a subject in need of treatment. In particular embodiments, they are administered as an active ingredient of a pharmaceutical composition. Pharmaceutical compositions of the present invention may comprise an effective amount of an agent that inhibits the hedgehog signaling pathway in combination with an agent that inhibits BCR-ABL, each as defined above, together with one or more acceptable carriers thereof. The compositions may also contain a third therapeutic agent noted above, e.g., a chemotherapeutic agent or other anti-cancer agent.

Pharmaceutical carriers enhance or stabilize the composition, or facilitate preparation of the composition. Pharmaceutically acceptable carriers are determined in part by the particular composition being administered (*e.g*., nucleic acid, protein, or other type of compounds), as well as by the particular method used to administer the composition. They should also be both pharmaceutically and physiologically acceptable in the sense of being compatible with the other ingredients and not injurious to the subject. They may take a wide variety of forms depending on the form of preparation desired for administration, e.g., oral, sublingual, rectal, nasal, or parenteral. For example, an antitumor compound may be complexed with carrier proteins such as ovalbumin or serum albumin prior to their administration in order to enhance stability or pharmacological properties.

There are a wide variety of suitable formulations of pharmaceutical compositions of the present invention (see, e.g., Remington: The Science and Practice of Pharmacy, Mack Publishing Co., 20^{th} ed., 2000). Without limitation, pharmaceutically acceptable carriers include syrup, water, isotonic saline solution, 5% dextrose in water or buffered sodium or ammonium acetate solution, oils, glycerin, alcohols, flavoring agents, preservatives, coloring agents starches, sugars, diluents, granulating agents, lubricants, and binders, among others. The carrier may also include a sustained release material such as glyceryl monostearate or glyceryl distearate, alone or with a wax.

The pharmaceutical compositions may be prepared in various forms, such as granules, tablets, pills, suppositories, capsules, suspensions, salves, lotions and the like. The concentration of therapeutically active compound in the formulation may vary from about 0.1 - 100% by weight. Therapeutic formulations are prepared by any methods well known in the art of pharmacy. See, e.g., Gilman et al., eds., Goodman and Gilman's: The Pharmacological Bases of Therapeutics , 8th ed., Pergamon Press, 1990; Remington: The Science and Practice of Pharmacy, Mack Publishing Co., 20th ed., 2000; Avis et al., eds., Pharmaceutical Dosage Forms: Parenteral Medications, published by Marcel Dekker, Inc., N.Y., 1993; Lieberman et al., eds., Pharmaceutical Dosage Forms: Tablets, published by Marcel Dekker, Inc., N.Y., 1990; and Lieberman et al., eds., Pharmaceutical Dosage Forms: Disperse Systems, published by Marcel Dekker, Inc., N.Y., 1990.

The therapeutic formulations may be delivered by any effective means that may be used for treatment. Depending on the specific antitumor agent to be administered, the suitable means include oral, nasal, pulmonary administration, or parenteral (including subcutaneous, intramuscular, intravenous and intradermal) infusion into the bloodstream. For parenteral administration, antitumor agents of the present invention may be formulated in a variety of ways. Aqueous solutions of the modulators may be encapsulated in polymeric beads, liposomes, nanoparticles or other injectable depot formulations known to those of skill in the art. Additionally, the compounds of the present invention may also be administered encapsulated in liposomes. The compositions, depending upon its solubility, may be present both in the aqueous layer and in the lipidic layer, or in what is generally termed a liposomic suspension. The hydrophobic layer, generally but not exclusively, comprises phospholipids such as lecithin and sphingomyelin, steroids such as cholesterol, more or less ionic surfactants such a diacetylphosphate, stearylamine, or phosphatidic acid, and/or other materials of a hydrophobic nature.

The therapeutic formulations may conveniently be presented in unit dosage form and administered in a suitable therapeutic dose. A suitable therapeutic dose may be determined by any well known methods such as clinical studies on mammalian species to determine maximum tolerable dose and on normal human subjects to determine safe dosage. Except under certain circumstances when higher dosages may be required, the dosage of an antitumor agent of the present invention usually lies within the range of from about 0.001 to about 1000 mg, more usually from about 0.01 to about 500 mg per day. The dosage and mode of administration of an antitumor agent may vary for different subjects, depending upon factors that may be individually reviewed by the treating physician, such as the condition or conditions to be treated, the choice of composition to be administered, including the particular antitumor agent, the age, weight, and response of the individual subject, the severity of the subject's symptoms, and the chosen route of administration. As a general rule, the quantity of an antitumor agent administered is the smallest dosage which effectively and reliably prevents or minimizes the conditions of the subjects. Therefore, the above dosage ranges are intended to provide general guidance and support for the teachings herein.

### EXAMPLES

The following examples are provided to illustrate the present invention. All animal experiments are in accordance with the US National Institutes of Health Statement of Compliance with Standards for Humane Care and Use of Laboratory Animals.

### Example 1

### General Materials and Methods

### Mice Experiments

Ptch+/- mice (Jackson Laboratory), Smo-/- mice (Deltagene), C57BL/6 mice (Jackson laboratory) and B6-Pep3b-*Ly5.1* (Pep) mice) are maintained and genotyped as described. For bone marrow transplantation experiments, C57BL/6 males are injected with 5-FU (150 mg/kg) intraperitoneal and sacrificed four days later. Bone marrow mononuclear cells are flushed from the leg bones, red blood cells are lysed with ammonium-chloride and bone marrow cells are cultivated in DMEM containing 10% FBS, SCF, IL-6 and IL-3. Cells are infected with a pMSCV/BCR-ABL/IRES/GFP retrovirus, 5 x 10⁵ mononuclear cells are transplanted into lethally irradiated C57BL/6 mice. Treatment with AMN107 50 mg/kg bid (Novartis, Basel) and cyclopamine 25 mg/kg bid (Novartis, Cambridge) started day 7 after transplantation for 14 days.

For transplantation experiments with Ptch and Smo hematopoietic cells, embryos day 14.5 of the gestation period are used. Embryos are chilled on ice and decapitated. The embryonic liver is extracted and liver cells are filtered through a cell strainer (BD Bioscience). Embryonic liver cells are either directly transplanted into sublethally irradiated B6-Pep3b-*Ly5.1* (Pep) mice for repopulation experiments, or cultured in stimulation media and then infected with a pMSCV/BCR-ABL/IRES/GFP retrovirus. The number of GFP positive cells is determined 24 hours after infection by flow cytometry, with the infection rate kept between 4-6% to evaluate the expansion of the BCR-ABL positive cells. Fetal liver cells are then transplanted into lethally irradiated recipients. Disease development is monitored by weekly weight measurements, biweekly blood cell counts and detection of GFP positive cells in the peripheral blood.

### Cell Culture Experiments

Bone marrow cells from diseased mice are cultured in DMEM media containing 10% FBS (Gibco), SCF (RDI), IL-3 and IL-6 (R&D systems). For in-vitro treatment experiments, 4 x 10⁶ bone marrow or spleen cells are seeded into 1 well of a 6-well plate. Cyclopamine-KAAD (obtained from Toronto Research Chemicals) is dissolved as x 1,000 stock in DMSO. After 72 hours of treatment, cells are plated in methyl cellulose media containing SCF, IL-6, IL-3 and insulin from stem cell technologies (M3434) according to the manufacturer's instruction. Colonies are counted 5 days and 10 days after plating. After 12 days, cells are diluted from the plates, washed in PBS and then either stained for analysis of different cell types or replated into a second or third plating round.

### Immunohistochemistry

Mouse tissue is fixed for at least 24 hrs, and paraffin embedded tissues are generated after standard procedure. Single color DAB-immunoperoxidase staining is performed on paraffin sections using antibodies to Gli1 (N-16, Santa Cruz Biotechnology), Smo (H-300, Santa Cruz Biotechnology) and Hh (H-160, Santa Cruz Biotechnology) according to the manufacturer's recommendation.

### RT-PCR and Quantitative PCR

RNA is extracted from CD34+ cells from CML patients in chronic phase or blast crisis of disease, from whole bone marrow or from sorted Lin-Kit+Sca+ positive cells using a Qiagen RNA extraction kit according to the manufacturer's recommendation. Quantitative PCR is assessed by Taqman PCR. Primers and probes are obtained from Applied Biosystems.

### Cell Staining and Sorting

Flow cytometry stainings for analysis of hematological cell types is performed using the antibodies Sca-PE, Kit-APC, Lin markers CD3, Gr-1, CD11b, CD19, Ter119 all PE-Cy7 positive, CD4-PE, CD8-APC from BD Pharmingen according to the manufacturer's instructions. For cell cycle analysis of stem cells, cells are treated with cyclopamine for 48 hours, then stained with Lin markers, Kit-APC and Sca-PE. Stained bone marrow is then fixed in 2% Formalin. Cells are permeabilized with 70% chilled ethanol for at least 1 hour and then treated with propidium iodide (5mg/ml) for at least 30 minutes. Cells are analyzed using a flow cytometer from Coulter. Annexin staining is performed after incubation of mixed bone marrow with cyclopamine for 24, 48 and 72 hours. Cells are stained with Annexin-PE antibody and 7-AAD (BD Bioscience) according to the manufacturer's instructions.

### Example 2

### Hedgehog Signaling Pathway Activation by BCR-ABL

As shown in this example, BCR-ABL activates the hedgehog signaling pathway in leukemic stem cells via upregulation of Smo. To evaluate the activation status of the hedgehog signaling pathway in BCR-ABL positive LSCs versus normal HSCs, the transcript levels of two Hh pathway target genes Gli1 and Ptch1 in human CD34+ cells from healthy donors to CD34+ cells isolated from patients with CML in chronic phase or blast crisis are compared. In all CML cases, a more than 4-fold induction of the transcript levels of Gli1 and Ptch1 is observed, indicating activation of the pathway in CML independent of the phase of the disease (**FIGURE 1A**). Gli1 and Ptch1 transcript levels are elevated in patients with CML blast crisis versus chronic phase of disease.

To further evaluate the effect of BCR-ABL on hedgehog pathway activation, a CML-like syndrome is induced in mice. Bone marrow infected with a pMSCV/ BCR-ABL /GFP virus is transplanted into irradiated recipient mice. BCR-ABL positive LSCs (Lin-Kit+Sca+GFP+) obtained from diseased mice displayed enhanced Gli1 and Ptch1 transcript levels compared to normal mouse HSCs (Lin-Kit+Sca+). The activation of the hedgehog pathway in mouse bone marrow infected with a BCR-ABL retrovirus (pMSCV) is not restricted to the stem cell population, but is present in all BCR-ABL overexpressing cells (**FIGURE 1B**).

An upregulation of the transmembrane receptor Smo is found in all BCR-ABL/GFP positive bone marrow cells versus much lower Smo levels in the BCR-ABL negative population in the same mouse. The upregulation of Smo in the BCR-ABL positive population could be detected by flow cytometry, as well as immunohistochemistry. IHC stainings from spleens and bone marrow of diseased mice with a Smo-specific antibody showed a strong induction of Smo expression in the BCR-ABL positive population. IHC stainings for Smo and Gli1 in human CML cases also revealed upregulation of both genes in corresponding regions of the bone marrow, especially in the blast cell population (**FIGURE 1C**). Furthermore, retroviral expression of Smo in lymphoma cells has been shown to facilitate the growth of Eµ-Myc positive lymphoma xenografts in non-lymphoid organs like the skin and enhances Gli1 level even in the absence of ligand stimulation.

### Example 3

### Inhibition of Hedgehog Signaling in vitro

This example shows that inhibition of hedgehog signaling in vitro induces apoptosis in BCR-ABL positive cells, and reduces the number of leukemic stem cells. To investigate the role of the hedgehog pathway in BCR-ABL positive bone marrow cells and leukemic stem cells in vitro, hedgehog signaling is inhibited by using KAAD-cyclopamine, an alkaloid which locks Smo in its inactive conformation. Bone marrow from mice with CML-like syndrome which contained about 50% BCR-ABL GFP positive cells versus 50% normal bone marrow cells is used. Cyclopamine treatment of mixed bone marrow cultures for three days resulted in a dose dependent reduction of the GFP/ BCR-ABL positive population compared to the GFP negative population. GFP positive cells after in vitro treatment with cyclopamine (2 µM or 5 µM) can be detected by flow cytometry analysis.

Further characterization of the different cell subsets showed a reduction of BCR-ABL positive myeloid progenitor cells (Lin-Kit+Sca-) by more than 80%, and a reduction of the Lin-Kit+Sca+ leukemic stem cell population by around 70% (**FIGURE 2A**). The main effect of cyclopamine inhibition on BCR-ABL positive bone marrow cells is apoptosis induction within 24 hours, measured by AnnexinV staining. Alterations in the cell cycle with a relative increase of the G1 phase compared to S phase and G2 phase in the complete bone marrow is also detected. Cell cycle analysis of the leukemic stem cell population showed a complete loss of the G2 phase in those cells after Hh pathway inhibition. Gli1 transcript levels in the bone marrow are reduced after treatment with cyclopamine, verifying the inhibition of the hedgehog signaling pathway in those cells by the compound (**FIGURE 2B**). In Figure 2B, bone marrow cultures are treated with either DMSO alone or different concentrations of cyclopamine (2 µM or 5 µM) for six hours. RNA is extracted from treated cultures and Glil transcript levels are measured by Taqman PCT and normalized to GAPDH. Assays are done in triplicates.

To further validate the effect of hedgehog pathway inhibition on the self renewing progenitor and leukemic stem cell population, mixed bone marrow and spleen cultures are treated with different concentrations of cyclopamine-KAAD (10, 5, 2.5, 1 and 0 uM) for 48 hours. The cells are then plated in methyl cellulose plates without supplementary cytokines, so that only BCR-ABL positive cells can survive. Colonies are counted 10 days after plating. Bone marrow and spleen cultures pretreated with cyclopamine showed a dose dependent reduction of BCR-ABL positive colonies, indicating that the colony forming ability of BCR-ABL positive cells is dependent on hedgehog pathway activation (**FIGURE 2C**).

### Example 4

### Hedgehog Pathway Activation

Hedgehog pathway activation enhances colony forming capacity and regeneration potential of hematopoietic progenitor and stem cells. To evaluate the role of hedgehog signaling in normal hematopoiesis, fetal HSCs are isolated from the liver of embryos day 14.5 of gestation period. Fetal liver cells from Smo^{-/-}, Smo^{+/-}, Smo^{+/+}, Ptch^{+/+} and Ptch^{+/-} embryos are analyzed regarding the number of fetal HSCs, number of differentiated hematopoietic cell types as well as colony forming capacity and repopulation potential in a transplantation experiment. No differences in the number of fetal HSCs between the different genotypes are found. There are also no significant differences in B-cells (B220), myeloid cells (CD11b) and erythroid progenitors (Ter119)) and CD3 positive T-cells.

Plating of cells into methyl cellulose agar with supplementary cytokines (IL-3, IL-6, SCF) did not result in any differences in the number of colonies, in the colony types or in the percentage of different cell types as measured by flow cytometry 10 days after plating. In contrast to the first plating round, big differences are observed in colony forming potentials in the second plating round. Replating Ptch and Smo wt hematopoietic cells showed only very limited colony forming potential in the second plating round, and Smo^{-/-} hematopoietic cells had lost the colony forming potential completely. In contrast, Ptch^{+/-} hematopoietic cells kept their ability to form colonies over more than 3 plating rounds, indicating that hedgehog pathway activation enhances the amount of regenerating cells in the Ptch^{+/-} hematopoietic population (Table 2).

**Table 2**

| Colony Numbers 10 d after plating of fetal livel cells (plating rounds P1-P3) | | | |
|---|---|---|---|
| **Genotype** | **P1** | **P2** | **P3** |
| Smo -/- | 130 | 0 | 0 |
| Smo +/- | 142 | 0 | 0 |
| Smo +/+ | 121 | 2 | 0 |
| Ptch +/+ | 128 | 3 | 0 |
| Ptch +/- | 136 | 48 | 23 |

In a second experiment, Smo^{-/-}, Smo^{+/-}, Smo^{+/+}, Ptch^{+/+} and Ptch^{+/-} fetal liver cells (positive for Ly-5.2) are transplanted into sublethally irradiated C57BL/6-Ly5.1-Pep 3b (B6 Ly-5.1) mice. The regeneration of Ly5.2 positive hematopoiesis in the peripheral blood showed a significant advantage for mice transplanted with the Ptch+/- fetal liver cells compared to the other transplanted fetal liver genotypes. The number of Ly5.2 positive cells in the peripheral blood is about doubled compared to wt and Smo-/- over a period from more than 3 months (**FIGURE 3A**). The regeneration of Smo-/- bone marrow is not significantly different from the wt, indicating that there are no big differences in the regeneration capacity of Smo-/- versus Smo wt HSCs. Further analysis of the cell types in the peripheral blood showed differences in the distribution of cells between mice transplanted with Smo-/- versus Smo wt fetal liver cells. Smo-/- showed a more than 90% decrease in CD8 positive T-cells, while the number of CD4+ T-cells is decreased only by 30%. These results show that hedgehog signaling is important for T-cell development, and indicate that the generation of CD8+ T-cells is dependent on intact hedgehog signaling (**FIGURE 3B**).

To further investigate the role of hedgehog signaling in HSCs, the regeneration capacity of the bone marrow of the mice, which are initially transplanted with the fetal liver cells, is investigated by injecting those mice with 5-fluorouracil (5-FU). The short term regeneration capacity is significantly reduced in bone marrow lacking Smo. Ten days after 5-FU injection, the number of Ly5.2 positive cells in mice initially transplanted with Smo-/- fetal liver cells are 70% lower than in the other genotypes indicating a role of the hedgehog signaling pathway in the short term repopulating cells (**FIGURE 3C**). These results show that Ptch +/mice display a faster regeneration potential in the short term repopulating cells and have a significantly enhanced stem cell pool. The results indicate that the long term repopulating cells profit from hedgehog pathway activation as the number of Ly5.2 positive cells in Ptch+/- mice stayed significantly higher than in the other genotypes for more than 3 months. The blood cell counts from two years old Ptch +/- mice show no difference in the number of peripheral blood cells compared to Ptch wt mice, indicating that there is no significant lack in the generation of blood cells in these mice even after a long period of time.

To further validate the role of upregulation of Smo in hematopoiesis, a GFP control vector, Smo wt and the activated mutant SmoW535E are overexpressed in the bone marrow of 5-FU pretreated mice. Irradiated donor mice are transplanted with 10% of GFP positive bone marrow cells mixed with 90% GFP negative bone marrow cells. Regeneration of hematopoiesis is monitored by blood cell counts and evaluation of GFP positive cells in the peripheral blood. Bone marrow cells overexpressing Smo wt or SmoW535E had significantly elevated Glil levels compared to control bone marrow cells (**FIGURE 3D**). The percentage of GFP positive cells in mice transplanted with bone marrow expressing the GFP control vector stayed between 10-12%. In contrast, mice transplanted with bone marrow infected with Smo wt or SmoW535T showed a significant increase in the number of GFP positive cells over one year to a maximum of 30%. There are no significant differences in the GFP positive cell types (**FIGURE 3E**). These data indicate that activation of the hedgehog signaling by overexpression of Smo can expand the stem cell pool, and significantly enhance the number of repopulating cells over time.

### Example 5

### Inhibition of BCR-ABL Positive Leukemic Stem Cells by Smo^{-/-} in vivo

As shown in this example, Smo^{-/-} inhibits expansion of BCR-ABL positive leukemic stem cells and abrogates retransplantability of the disease. To investigate the role of the hedgehog pathway in the development of BCR-ABL positive leukemias *in vivo,* BCR-ABL is overexpressed in Smo^{-/-}, Smo^{+/-}, Smo^{+/+}, Ptch^{+/+} and Ptch^{+/-} embryonic liver cells using a pMSCV/ BCR-ABL /IRES/GFP retroviral vector. The infection rate is between 3-4% in all tested embryonic hematopoietic cells. Infected cells are transplanted into irradiated recipient C57/B16 mice. GFP positive cells and blood cell counts are measured 20 days after transplantation. Mice transplanted with Ptch^{+/-}/BCR-ABL/GFP fetal liver cells showed 3-fold higher GFP levels than mice transplanted with Ptch wt or Smo wt bone marrow infected with pMSCV/ BCR-ABL /GFP. Smo^{-/-}/BCR-ABL/GFP positive cells did not expand in this time span and showed even numbers below the original infection rate (**FIGURE 4A**). Day 28 after transplantation, three mice are taken from each transplantation group, and the spleen weights between the different groups are compared.

All mice transplanted with Ptch^{+/-}, Ptch wt, Smo wt or Smo^{+/-}/BCR-ABL /GFP fetal liver cells had a more than 40% increase in spleen weight as a sign of starting CML development, while all mice transplanted with Smo-/- embryonic liver cells had a normal spleen size, indicating that Smo is important for the expansion of the BCR-ABL positive cells (**FIGURE 4B**). All mice transplanted with the Ptch^{+/-} embryonic liver cells developed a lethal leukemic disease within 38 days after transplantation, followed by mice transplanted with Ptch wt, Smo wt or Smo^{+/-} fetal liver cells (**FIGURE 4C**). Mice transplanted with Ptch^{+/-} fetal liver cells are more likely to develop BCR-ABL positive ALLs (80%) than CMLs (20%), while mice transplanted with Smo+/- fetal liver cells are more likely to develop CMLs than ALLs. Only 60% of the mice transplanted with Smo^{-/-} BCR-ABL positive fetal liver cells developed a lethal disease more than 3 months after transplantation, which is characterized by enhanced spleen weight but none of the mice showed enhanced white blood cell counts in the peripheral blood. Forty percent of the Smo^{-/-}/ BCR-ABL /GFP transplanted mice did not show any signs of disease even 12 months after transplantation.

To further investigate the activation status of the hedgehog signaling pathway on the leukemic stem cell population, bone marrow and spleen cells are collected from the diseased mice from the first infection round, and 2E5 GFP positive cells are transplanted into irradiated secondary recipients. All secondary recipients from mice transplanted with Ptch^{+/-}, Ptch wt, Smo wt and Smo^{+/-} BCR-ABL positive bone marrow developed leukemias within 2 months after transplantation, while none of the mice transplanted with Smo^{-/-} BCR-ABL wt bone marrow developed any signs of disease even 4 months after transplantation (**FIGURE 4D**). These results indicate that the expansion of the BCR-ABL positive leukemic stem cell is dependent on hedgehog pathway activation, and that Smo may be a target for leukemic stem cells in CML.

### Example 6

### Combination of Abl Inhibition and Smo Inhibition In Vivo

As shown in this example, the combination of Abl inhibition (e.g., AMN107) and Smo inhibition (e.g., cyclopamine) in mice with CML-like disease reduces the amount of colony forming units and enhances time to relapse, indicating that the combination of AMN107 and cyclopamine may be beneficial in the treatment of CML.

Mice transplanted with BCR-ABL positive bone marrow is treated with either a suboptimal dose of the ABL inhibitor AMN107, or with a combination of AMN107 (50 mg/kg qd) and the Smo antagonist cyclopamine (25 mg/kg bid). Treatment is started seven days after transplantation and is continued for fourteen days total. At the end of the treatment, three mice in each group are sacrificed and bone marrow from 1 femur is plated in methyl cellulose colony assays without addition of cytokines to detect only BCR-ABL positive colonies. The average number of colonies detected in mice treated with the combination AMN107 and cyclopamine is reduced more than 40% compared to the AMN107 only treatment group, indicating that the combination treatment can reduce the number of BCR-ABL positive colony forming units (FIGURE 5A). Peripheral blood cell counts, spleen and liver weights are normal at that time point, and the number of GFP positive cells is below 5%.

Eight days after the end of treatment, another three mice per group are sacrificed and examined for signs of relapse by comparing liver and spleen weight. Enhanced liver and spleen weight are found in all mice compared to normal mice, but mice treated with AMN107 alone had about double the average spleen size and a much higher liver weight than the mice treated with the combination of AMN107 and cyclopamine (**FIGURE 4B**). The five remaining mice in each group are monitored for signs of disease and sacrificed when moribund. The average survival after end of treatment in the AMN107 group alone is eight days versus 24 days in the AMN107 and cyclopamine treatment group (**FIGURE 5C**).

## Claims

1. A combination of a first agent that inhibits hedgehog signaling pathway wherein said first agent is cyclopamine or forskolin and a second agent that inhibits BCR-ABL wherein said second agent is selected from the group consisting of

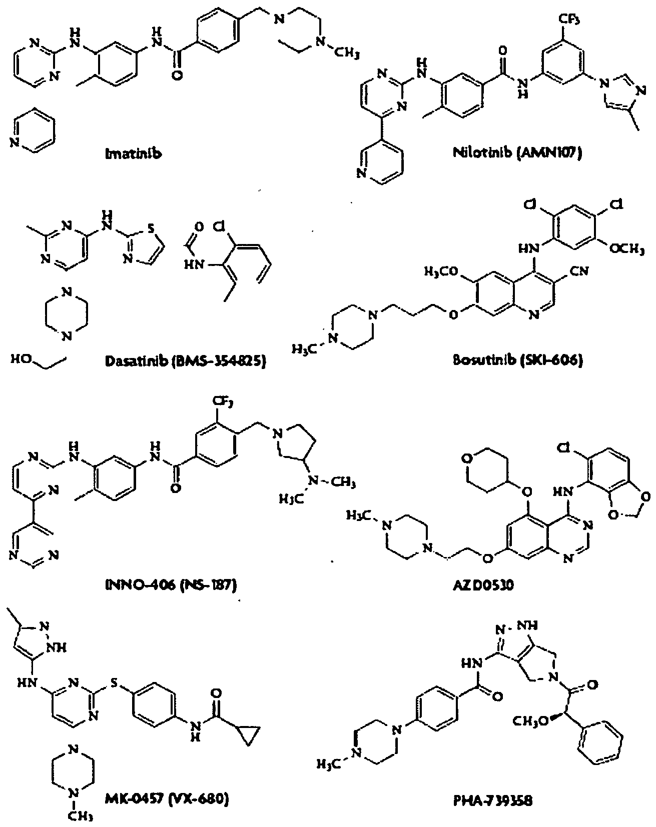

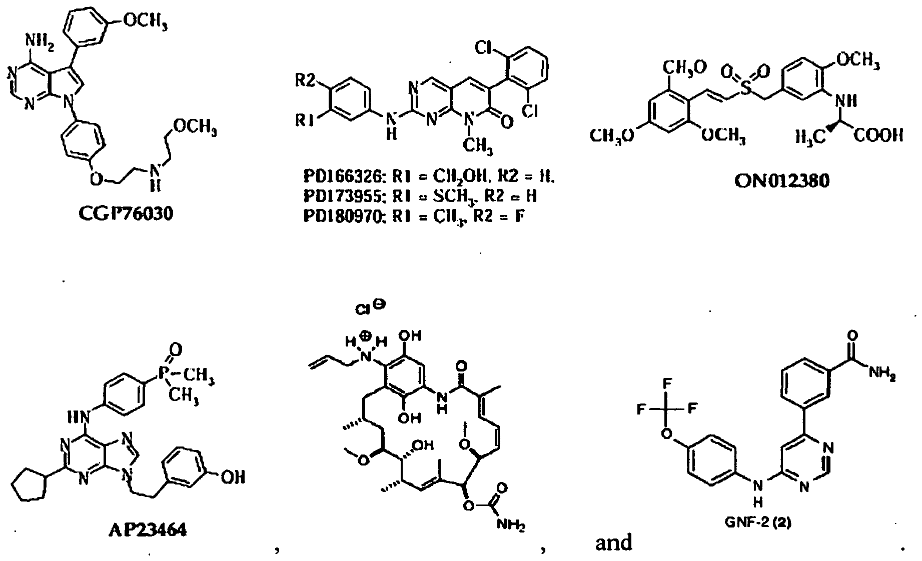

2. A composition comprising a first agent that inhibits hedgehog signaling pathway wherein said first agent is cyclopamine or forskolin and a second agent that inhibits BCR-ABL wherein said second agent is selected from the group consisting of

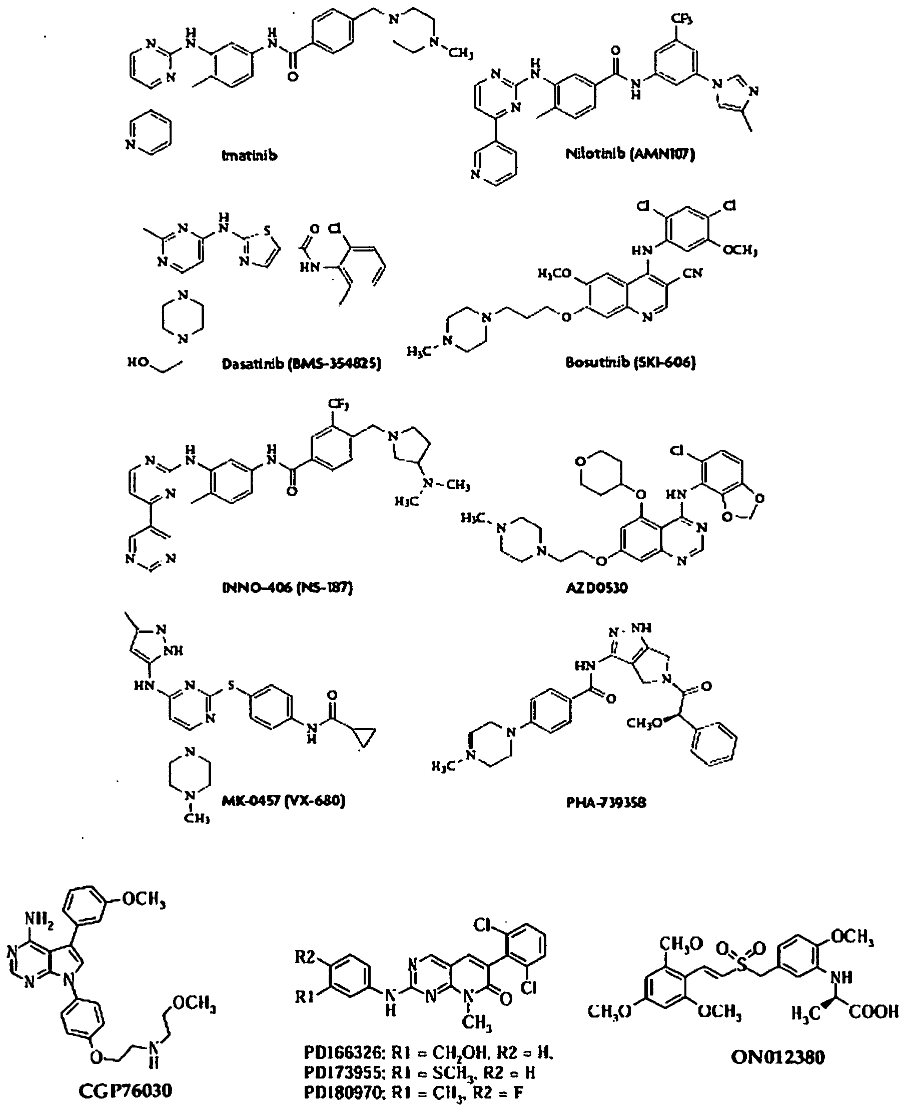

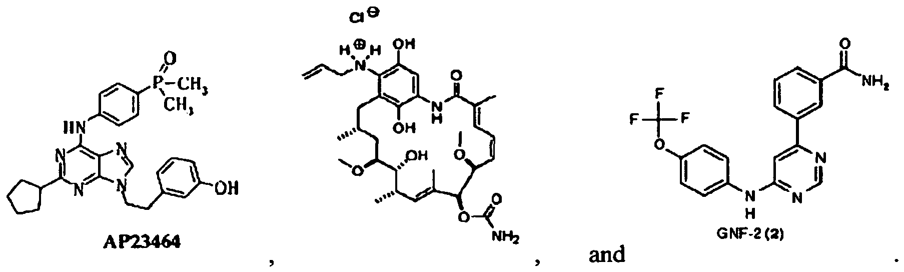

3. The composition of claim 2 wherein said first agent binds to Smo.

4. The pharmaceutical composition of claim 2 and a pharmaceutically acceptable carrier.

5. The use of a composition of any one of claims 2-4, for the manufacture of a medicament for treating BCR-ABL positive leukemia.

6. The use of claim 5, wherein said BCR-ABL positive leukemia is chronic myeloid leukemia or acute lymphocyte leukemia.

7. The combination of claim 1 or the composition of any one of claims 2-4 for use in the treatment of BCR-ABL positive leukemia.

8. The combination or the composition of claim 7 wherein said BCR-ABL positive leukemia is chronic myeoloid leukemia or actue lymphocyte leukemia.

## Patentansprüche

1. Kombination eines ersten Mittels, das den Hedgehog-Signalweg hemmt, wobei das genannte erste Mittel Cyclopamin oder Forskolin ist, und eines zweiten Mittels, das BCR-ABL hemmt, wobei das zweite Mittel aus der Gruppe ausgewählt ist, die aus: 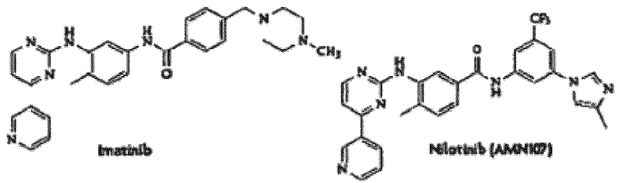 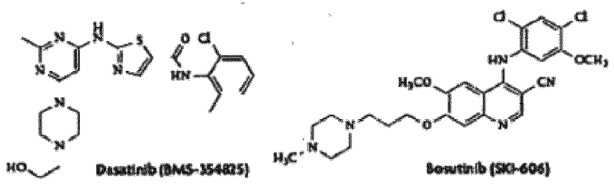 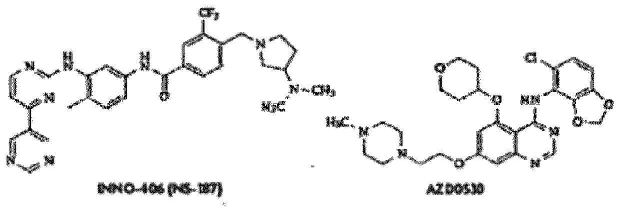 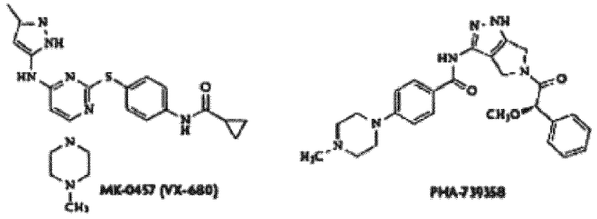 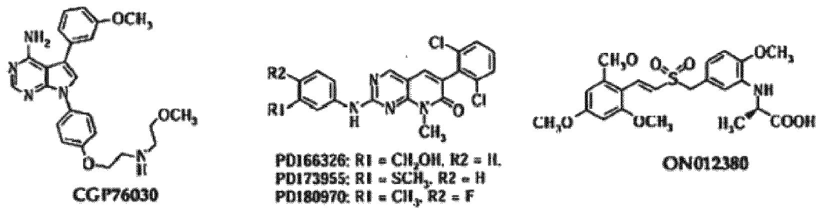 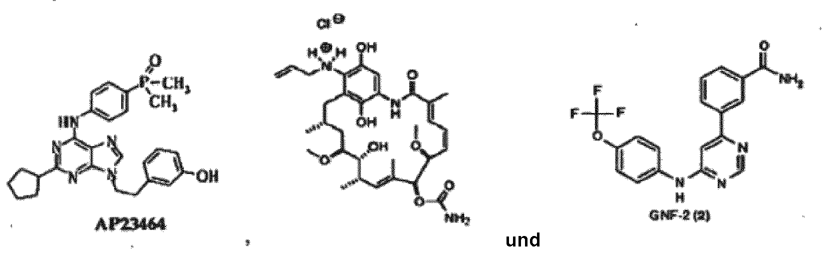 besteht.

2. Zusammensetzung, umfassend ein erstes Mittel, das den Hedgehog-Signalweg hemmt, wobei das erste Mittel Cyclopamin oder Forskolin ist, und ein zweites Mittel, das BCR-ABL hemmt, wobei das genannte zweite Mittel aus der Gruppe ausgewählt ist, die aus: 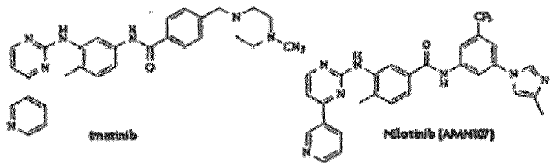 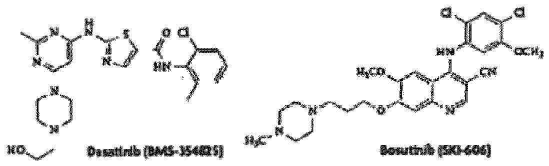 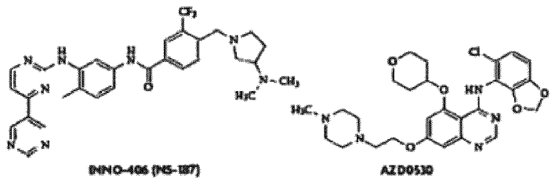 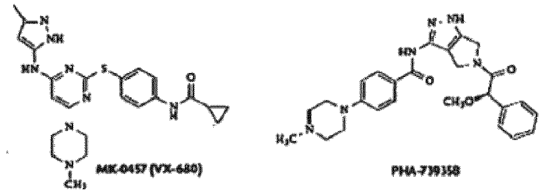 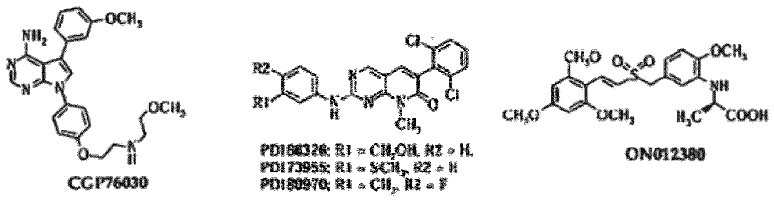 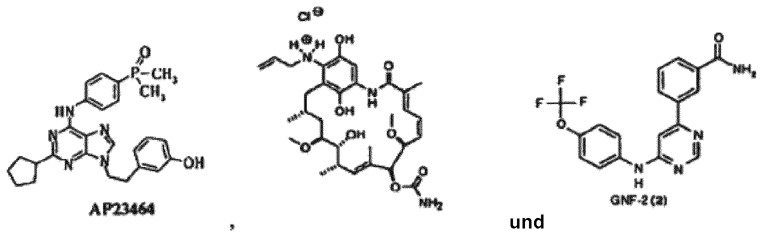 besteht.

3. Zusammensetzung nach Anspruch 2, wobei das genannte erste Mittel an Smo bindet.

4. Pharmazeutische Zusammensetzung nach Anspruch 2 und ein pharmazeutisch akzeptabler Träger.

5. Verwendung einer Zusammensetzung nach einem der Ansprüche 2 bis 4 zur Herstellung eines Medikaments zum Behandeln zum BCR-ABL-positiver Leukämie.

6. Verwendung nach Anspruch 5, wobei es sich bei der genannten BCR-ABL-positiven Leukämie um chronische myeloische Leukämie oder akute lymphatische Leukämie handelt.

7. Kombination nach Anspruch 1 oder Zusammensetzung nach einem der Ansprüche 2 bis 4 zur Verwendung bei der Behandlung von BCR-ABL-positiver Leukämie.

8. Kombination oder Zusammensetzung nach Anspruch 7, wobei die genannte BCR-ABL-positive Leukämie chronische myeloische Leukämie oder akute lymphatische Leukämie ist.

## Revendications

1. Combinaison d'un premier agent qui inhibe la voie de transmission du signal Hedgehog, ledit premier agent étant la cyclopamine ou la forskoline, et d'un deuxième agent qui inhibe BCR-ABL, ledit deuxième agent étant choisi dans le groupe constitué par

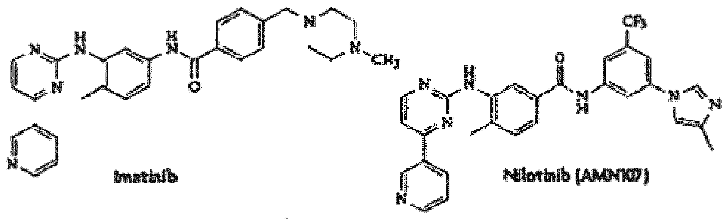

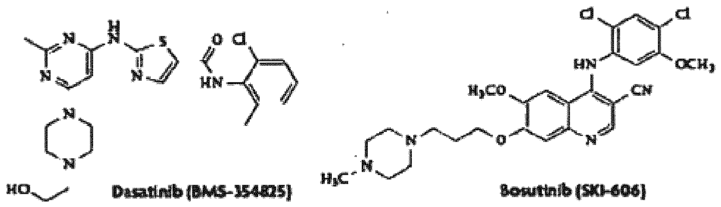

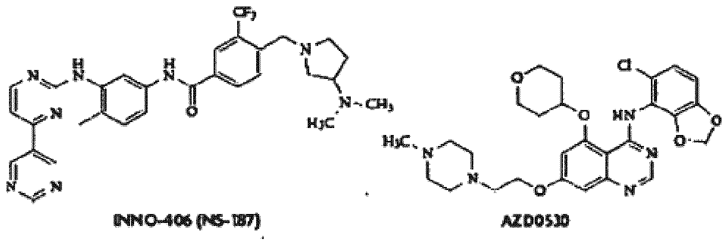

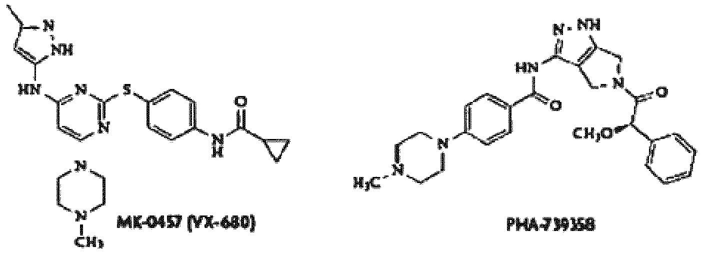

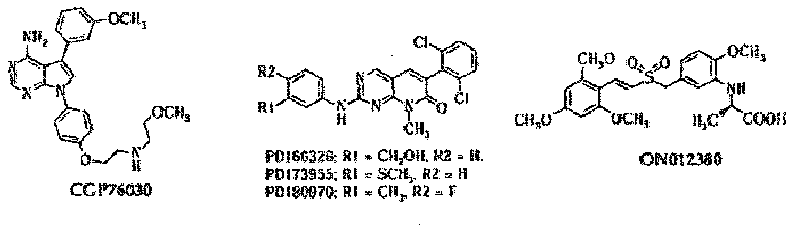

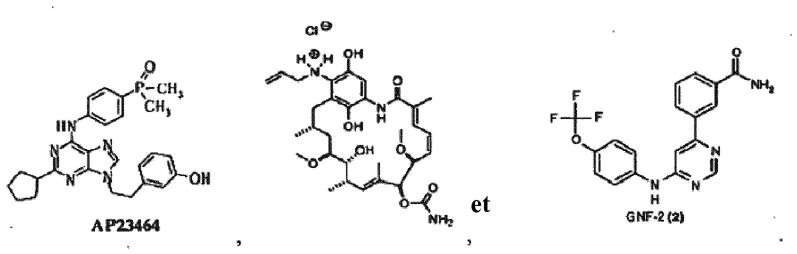

2. Composition comprenant un premier agent qui inhibe la voie de transmission du signal Hedgehog, ledit premier agent étant la cyclopamine ou la forskoline, et un deuxième agent qui inhibe BCR-ABL, ledit deuxième agent étant choisi dans le groupe constitué par

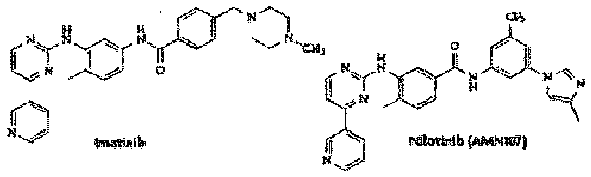

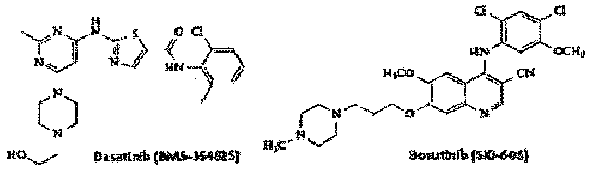

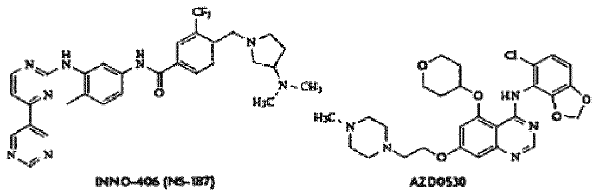

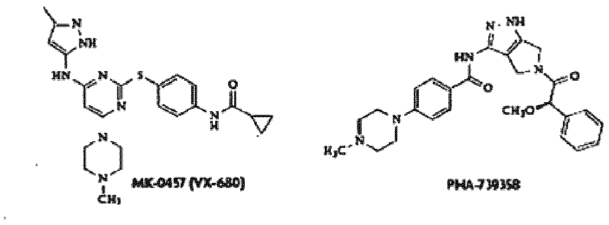

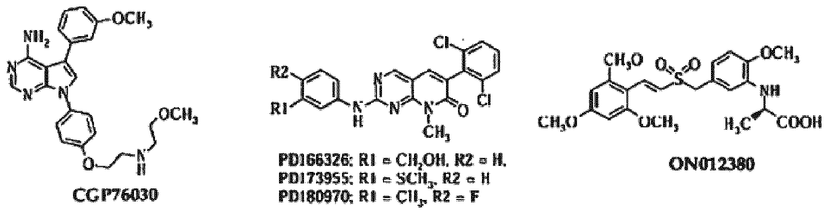

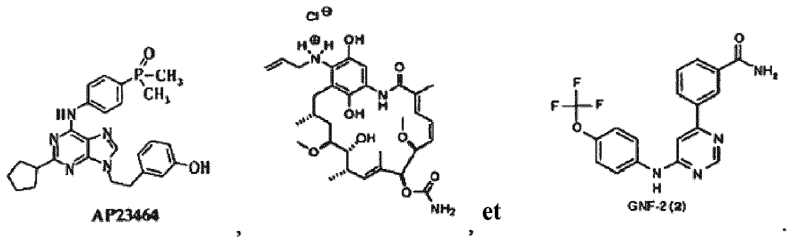

3. Composition selon la revendication 2, dans laquelle ledit premier agent se lie à Smo.

4. Composition pharmaceutique selon la revendication 2 et un véhicule acceptable sur le plan pharmaceutique.

5. Utilisation d'une composition selon l'une quelconque des revendications 2-4, pour la fabrication d'un médicament destiné au traitement d'une leucémie BCR-ABL-positive.

6. Utilisation selon la revendication 5, où ladite leucémie BCR-ABL-positive est la leucémie myéloïde chronique ou la leucémie lymphoblastique aiguë.

7. Combinaison selon la revendication 1 ou composition selon l'une quelconque des revendications 2-4, destinée à être utilisée dans le traitement d'une leucémie BCR-ABL-positive.

8. Combinaison ou composition selon la revendication 7, où ladite leucémie BCR-ABL-positive est la leucémie myéloïde chronique ou la leucémie lymphoblastique aiguë.
